(19) Europäisches Patentamt
European Patent Office
Office européen des brevets

(11) EP 4 741 371 A1

(12) EUROPEAN PATENT APPLICATION
published in accordance with Art. 153(4) EPC

(43) Date of publication:
**13.05.2026 Bulletin 2026/20**

(21) Application number: **24836044.8**

(22) Date of filing: **02.07.2024**

(51) International Patent Classification (IPC):
***C07C 51/43*** *(2006.01)* ***C07C 27/02*** *(2006.01)*
***C07C 51/48*** *(2006.01)* ***C07C 59/66*** *(2006.01)*
***C08G 63/64*** *(2006.01)* ***C08J 11/16*** *(2006.01)*

(52) Cooperative Patent Classification (CPC):
(C-Sets available)
**C07C 51/43; C08G 63/64; C08J 11/16** (Cont.)

(86) International application number:
**PCT/JP2024/023955**

(87) International publication number:
**WO 2025/009531 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.07.2023 JP 2023109630**

(71) Applicant: **MITSUBISHI GAS CHEMICAL COMPANY, INC.**
**Chiyoda-ku**
**Tokyo 100-8324 (JP)**

(72) Inventors:
- **KATO Noriyuki**
**Tokyo 100-8324 (JP)**
- **NISHIMORI Katsushi**
**Tokyo 125-8601 (JP)**
- **MOTEGI Atsushi**
**Tokyo 125-8601 (JP)**
- **ISHIHARA Kentaro**
**Tokyo 100-8324 (JP)**
- **MATSUMOTO Mutsumi**
**Tokyo 125-8601 (JP)**
- **TAKAMATSU Kazutaka**
**Tokyo 125-8601 (JP)**
- **SATO Atsuhiro**
**Tokyo 125-8601 (JP)**

(74) Representative: **Hoffmann Eitle**
**Patent- und Rechtsanwälte PartmbB**
**Arabellastraße 30**
**81925 München (DE)**

# (54) METHOD FOR PRODUCING DICARBOXYLIC ACID COMPOUND AND METHOD FOR PRODUCING RECYCLED RESINS

(57) The present invention addresses the problem of efficiently recycling waste resin compositions such as synthetic resins, enabling the reuse of a wider variety of resins than before. The problem is solved by a method for producing a dicarboxylic acid compound, said method including a crystallization step for crystallizing at least the dicarboxylic acid compound from a mixed liquid containing a dicarboxylic acid compound represented by general formula (1-1) or general formula (1-2) and a dihydroxy compound represented by general formula (2-1) or general formula (2-2). The substituents and the like in the general formulas are as described in the description of the present application.

EP 4 741 371 A1

(52) Cooperative Patent Classification (CPC): (Cont.)

C-Sets
**C07C 51/43, C07C 59/70**

## Description

TECHNICAL FIELD

The present invention relates to a method for producing a dicarboxylic acid compound, a method for producing a recycled resin, and the like.

BACKGROUND ART

In recent years, there have been growing concerns about the deterioration of the natural environment and the increase in discharged wastes, and the movement to reuse and recycle plastic products has been further fueled in an effort to realize a recycling-oriented society.

Synthetic resins, such as polycarbonate resins, are major components of plastic products and have been widely used in various applications, including home appliances, electronic and electrical equipment, office automation equipment, optical media, automotive parts, and construction components. Since large amounts of waste materials from the synthetic resins are discharged during the manufacture of the plastic products and after the use of the plastic products, these waste materials are reused.

In particular, when a synthetic resin is molded to manufacture a plastic product, the portions originating from the mold passages, such as the sprue, runner, and gate, are removed to form the plastic product. Efforts have been made to recycle, rather than dispose, such an unwanted synthetic resin removed from the plastic product, as well as other waste resins such as defective molded products, to reuse them in products.

For example, Patent literature 1 describes a method for recovering a polycarbonate resin, the method comprising a step of comminuting waste optical discs and/or recovered optical discs having a polycarbonate resin substrate and chemically treating the resulting comminuted material. In such a recovery method, the chemically treated product obtained in the chemical treatment step undergoes the steps of: removing magnetic metallic foreign matter using a magnet, removing colored foreign matter using an optical camera, and removing a resin containing metallic foreign matter using a metallic foreign matter detector.

There are also known methods for recycling waste resin compositions and reusing compounds that have been used as resin feedstocks (e.g. Patent literature 2).

PRIOR ART DOCUMENTS

PATENT DOCUMENTS

Patent literature 1: Japanese Patent Publication No. 2011-131507
Patent literature 2: International Publication WO2023/058599

SUMMARY OF THE INVENTION

PROBLEMS TO BE SOLVED BY THE INVENTION

Among the conventional methods for recovering synthetic resins, for example, in the method described in Patent literature 1, the synthetic resin is recovered, for example, based on the metal contained or the appearance of the coloration. However, if there is no significant difference in the presence or absence of metal content, degree of coloration, etc. between the desired synthetic resin and other unwanted organic impurities, efficient recovery of the desired synthetic resin may be difficult.

Additionally, the types of resins that can be recovered and reused are limited in the conventional methods for recycling waste resin compositions.

Under such circumstances, there is a need for a new method for efficiently recycling a waste resin composition and reusing a wider variety of resins than before.

Therefore, the main purpose of the present invention is to provide a method for recycling a waste resin composition containing a synthetic resin to enable the reuse of a wider variety of resins than before.

MEANS FOR SOLVING THE PROBLEMS

The present invention has, for example, the following aspects.

[1] A method for producing a dicarboxylic acid compound represented by general formula (1-1) below or general formula (1-2) below, the method comprising:

a crystallization step in which at least the dicarboxylic acid compound is crystallized from a liquid mixture containing the dicarboxylic acid compound and a dihydroxy compound represented by general formula (2-1) below or general formula (2-2) below:

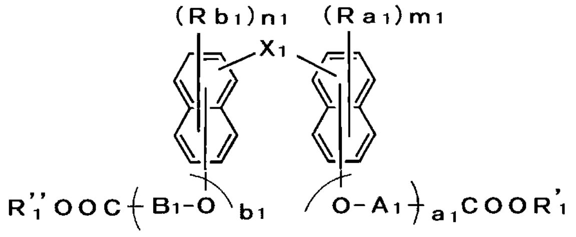

(1－1)

in general formula (1-1),
$R_{a1}$ and $R_{b1}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h1}$,
$R_{h1}$ represents an optionally substituted aryl group with 6-20 carbon atoms or an optionally substituted heteroaryl group with 6-20 carbon atoms containing one or more heteroatoms selected from O, N, and S,
$X_1$ represents a single bond or an optionally substituted fluorene group,
$A_1$ and $B_1$ each independently represent an optionally substituted alkylene group with 1-5 carbon atoms,
$m_1$ and $n_1$ each independently represent an integer from 0 to 6,
$a_1$ and $b_1$ each independently represent an integer from 0 to 10, and
$R_1$' and $R_1$" are each independently selected from the group consisting of a hydrogen atom, an optionally substituted alkyl group with 1-20 carbon atoms, and an optionally substituted aryl group with 6-20 carbon atoms;

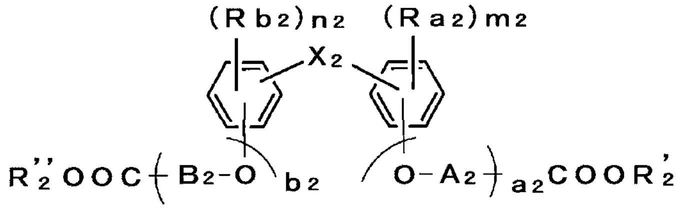

(1－2)

in general formula (1-2),
$R_{a2}$ and $R_{b2}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h2}$,
$R_{h2}$ represents an optionally substituted aryl group with 6-20 carbon atoms or an optionally substituted heteroaryl group with 6-20 carbon atoms containing one or more heteroatoms selected from O, N, and S,
$X_2$ is a single bond, an optionally substituted fluorene group, or any of the structural formulae represented by formulae (8)-(15) below:

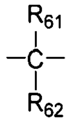

(8)

$-S-$

(9)

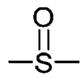

(10)

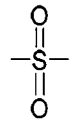

(11)

$-(CH_2)_r-$

(12)

$-O-$

(13)

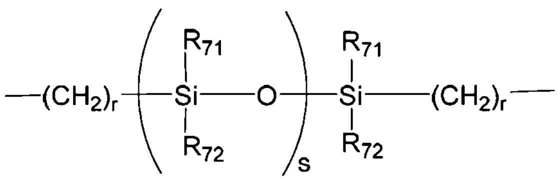

(14)

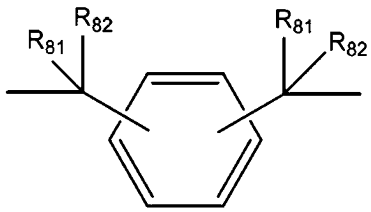

(15)

in formulae (8)-(15),

$R_{61}$, $R_{62}$, $R_{71}$, $R_{72}$,, $R_{81}$, and $R_{82}$ each independently represent a hydrogen atom, a halogen atom, an optionally substituted alkyl group with 1-20 carbon atoms, or an optionally substituted aryl group with 6-30 carbon atoms, or $R_{61}$ and $R_{62}$ or $R_{71}$ and $R_{72}$ bond to each other to form an optionally substituted carbon or heterocyclic ring with 1-20 carbon atoms,

$A_2$ and $B_2$ each independently represent an optionally substituted alkylene group with 1-5 carbon atoms,

$m_2$ and $n_2$ each independently represent an integer from 0 to 6,

$a_2$ and $b_2$ each independently represent an integer from 0 to 10, and

$R_2$' and $R_2$" are each independently selected from the group consisting of a hydrogen atom, an optionally substituted alkyl group with 1-20 carbon atoms, and an optionally substituted aryl group with 6-20 carbon atoms;

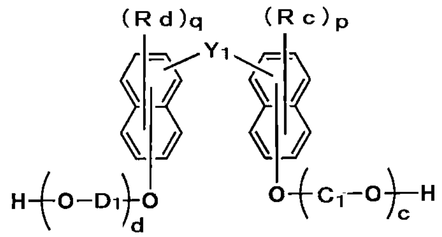

(2-1)

in general formula (2-1),

$R_c$ and $R_d$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C \equiv C-R_{h3}$,

$R_{h3}$ represents an optionally substituted aryl group with 6-20 carbon atoms or an optionally substituted heteroaryl group with 6-20 carbon atoms containing one or more heteroatoms selected from O, N, and S,

$Y_1$ represents a single bond or an optionally substituted fluorene group,

$C_1$ and $D_1$ each independently represent an optionally substituted alkylene group with 1-5 carbon atoms,

p and q each independently represent an integer from 0 to 6, and

c and d each independently represent an integer from 0 to 10; and

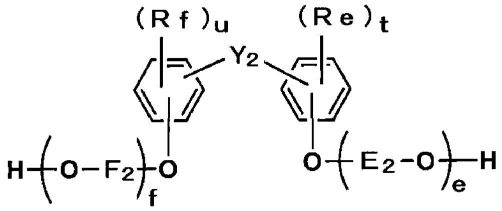

(2-2)

in general formula (2-2),

$R_e$ and $R_f$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms,

$Y_2$ is a single bond, an optionally substituted fluorene group, or any of the structural formulae represented by formulae (8)-(15) below:

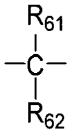 (8)

$-S-$ (9)

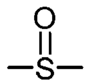 (10)

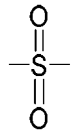 (11)

$-(CH_2)_r-$ (12)

$-O-$ (13)

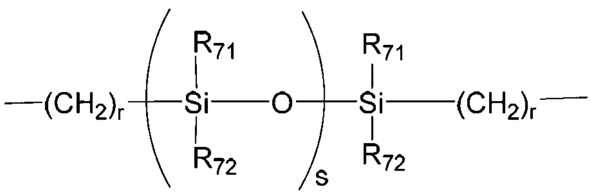

(14)

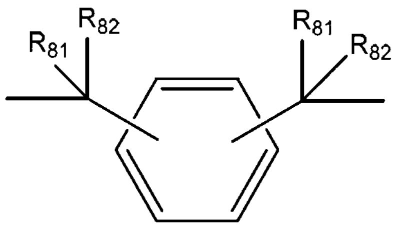

(15)

in formulae (8)-(15),

$R_{61}$, $R_{62}$, $R_{71}$, $R_{72}$, $R_{81}$, and $R_{82}$ each independently represent a hydrogen atom, a halogen atom, an optionally substituted alkyl group with 1-20 carbon atoms, or an optionally substituted aryl group with 6-30 carbon atoms, or $R_{61}$ and $R_{62}$ or $R_{71}$ and $R_{72}$ bond to each other to form an optionally substituted carbon or heterocyclic ring with 1-20 carbon atoms, and

r and s each independently represent an integer from 0 to 5,000,

$E_1$ and $F_1$ each independently represent an optionally substituted alkylene group with 1-5 carbon atoms,

t and u each independently represent an integer from 0 to 4, and

e and f each independently represent an integer from 0 to 10.

[2] The method for producing a dicarboxylic acid compound according to [1] above, further comprising a first washing step in which the liquid mixture containing the dicarboxylic acid compound is washed with an acidic aqueous solution.

[3] The method for producing a dicarboxylic acid compound according to [1] or [2] above, e.g., [1] above, further comprising a first solution forming step in which the dicarboxylic acid compound is dissolved in a polar solvent to obtain the liquid mixture.

[4] The method for producing a dicarboxylic acid compound according to [3] above, wherein

in the first solution forming step, the dicarboxylic acid compound and the dihydroxy compound are dissolved in a polar solvent to obtain the liquid mixture, and
in the crystallization step, a crystal of only the dicarboxylic acid compound is crystallized from the liquid mixture.

[5] The method for producing a dicarboxylic acid compound according to any one of [1]-[4] above, e.g., [1] above, further comprising a second washing step in which the liquid mixture containing the dicarboxylic acid compound is washed with an organic solvent.
[6] The method for producing a dicarboxylic acid compound according to any one of [1]-[5] above, e.g., [1] above, further comprising a second solution forming step in which the dicarboxylic acid compound is dissolved in an organic solvent to obtain the liquid mixture.
[7] The method for producing a dicarboxylic acid compound according to [6] above, wherein

in the second solution forming step, the dicarboxylic acid compound and the dihydroxy compound are dissolved in an organic solvent to obtain the liquid mixture, and
in the crystallization step, a co-crystal of the dicarboxylic acid compound and the dihydroxy compound is crystallized from the liquid mixture.

[8] The method for producing a dicarboxylic acid compound according to any one of [1]-[7] above, e.g., [1] above, further comprising a decomposition step in which a polyester carbonate resin and/or a polyester resin, which has at least a structural unit derived from a dicarboxylic acid compound represented by general formula (1-1') below or general formula (1-2') below, is decomposed to obtain the dicarboxylic acid compound represented by general formula (1-1) above or general formula (1-2) above:

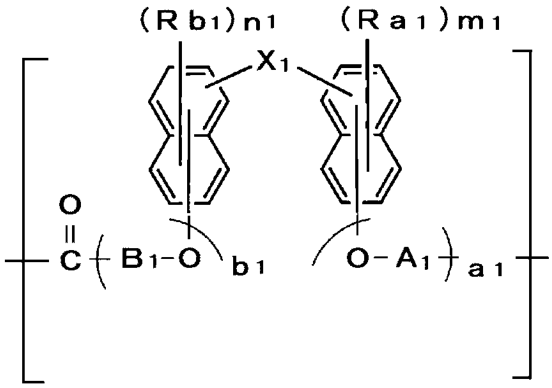

(1-1')

each symbol in general formula (1-1') is synonymous with the one in general formula (1-1) above, and

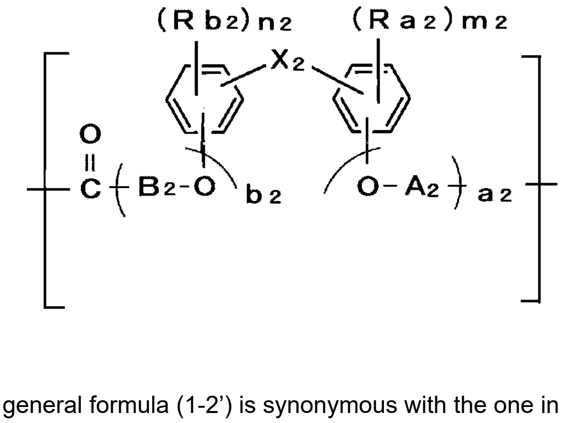

(1-2')

each symbol in general formula (1-2') is synonymous with the one in general formula (1-2) above.

[9] The method for producing a dicarboxylic acid compound according to [8] above, wherein

in the decomposition step, the polyester carbonate resin and/or the polyester resin is dissolved in a first solvent

and treated with an alkaline solution to obtain a reaction solution containing the first solvent and the dicarboxylic acid compound represented by formula (1-1) above or general formula (1-2) below,

in the crystallization step, the dicarboxylic acid compound is crystallized from a liquid mixture subjected to crystallization which is obtained by adding a second solvent to the reaction solution, and

the difference between the solubility of the dicarboxylic acid compound in the first solvent at 25°C and the solubility of the dicarboxylic acid compound in the second solvent at 25°C is 0.1 g/10 mL or more.

[10] The method according to [9] above, wherein the content of the first solvent in the liquid mixture subjected to crystallization is 0.1-10 g/g relative to the total amount of the mixture of the dicarboxylic acid compound.
[11] The method according to [9] or [10] above, e.g., [9] above, wherein the content of the second solvent in the liquid mixture subjected to crystallization is 0.3-3 g/g relative to the total amount of the mixture of the dicarboxylic acid compound.
[12] The method according to any one of [9]-[11] above, e.g., [9] above, wherein the total content of the first and second solvents in the liquid mixture subjected to crystallization is 1-10 g/g relative to the total amount of the dicarboxylic acid compound.
[13] The method according to any one of [9]-[12] above, e.g., [9] above, wherein the ratio of the content of the first solvent (g/g) to the content of the second solvent (g/g) (first solvent/second solvent) in the liquid mixture subjected to crystallization is 0.8-10.
[14] The method according to any one of [1]-[13] above, e.g., [1] above, wherein, in the crystallization step, a seed crystal of the dicarboxylic acid compound or the dihydroxy compound is added to the liquid mixture to crystallize a co-crystal of the dicarboxylic acid compound and the dihydroxy compound.
[15] A method for producing a dicarboxylic acid compound represented by general formula (1-1) below or general formula (1-2) below, the method comprising:

a crystallization step in which at least the dicarboxylic acid compound is crystallized from a liquid mixture containing the dicarboxylic acid compound,

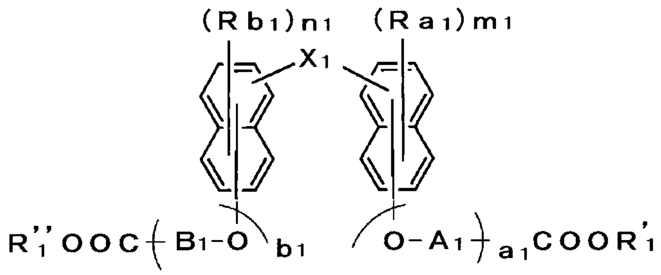

(1-1)

each symbol in general formula (1-1) is synonymous with the one in general formula (1-1) in [1] above; and

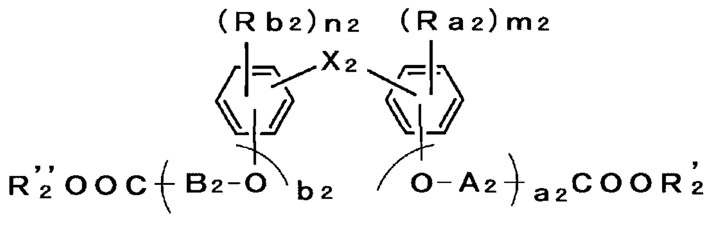

(1-2)

each symbol in general formula (1-2) is synonymous with the one in general formula (1-1) in [1] above.

[16] A method for producing a recycled resin, comprising a polymerization step in which a dicarboxylic acid compound produced by the method according to any one of [1]-[15] above, e.g., [1] above, is polymerized.

ADVANTAGEOUS EFFECT OF THE INVENTION

According to the present invention, a waste resin composition containing a synthetic resin can be recycled efficiently and a wider variety of resins can be reused than before.

DESCRIPTION OF EMBODIMENTS

Hereinafter, embodiments for carrying out the present invention will be described in detail.

1. Method for producing dicarboxylic acid compound

1-1. Dicarboxylic acid compound

According to one embodiment of the present invention, there is provided a method for producing a dicarboxylic acid compound represented by general formula (1-1) below or general formula (1-2) below. These dicarboxylic acid compounds are crystallized from a liquid mixture containing at least the dicarboxylic acid compound, and preferably from a liquid mixture containing a predetermined dihydroxy compound together with the dicarboxylic acid compound.

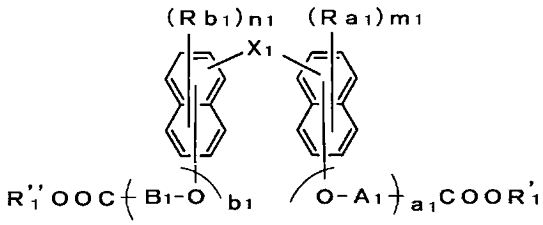

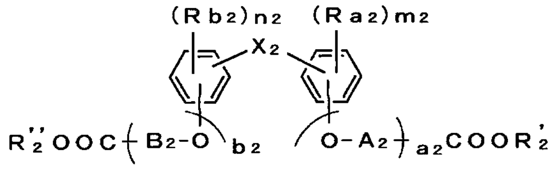

(1－1) (1－2)

In general formula (1-1), $R_{a1}$ and $R_{b1}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h1}$.

$R_{h1}$ is an optionally substituted aryl group with 6-20 carbon atoms, or an optionally substituted heteroaryl group with 6-20 carbon atoms containing one or more heteroatoms selected from O, N, and S. Preferably, $R_{h1}$ is an optionally substituted aryl group with 6-20 carbon atoms, and more preferably an optionally substituted aryl group with 6-12 carbon atoms.

$R_{a1}$ and $R_{b1}$ in general formula (1-1) are each preferably a hydrogen atom, an optionally substituted aryl group with 6-20 carbon atoms, or an optionally substituted heteroaryl group with 6-20 carbon atoms containing one or more heteroatoms selected from O, N, and S, more preferably a hydrogen atom or an optionally substituted aryl group with 6-20 carbon atoms, and still more preferably a hydrogen atom or an optionally substituted aryl group with 6-12 carbon atoms.

In general formula (1-1), $X_1$ represents a single bond or an optionally substituted fluorene group, preferably a single bond.

In general formula (1-1), $A_1$ and $B_1$ each independently represent an optionally substituted alkylene group with 1-5 carbon atoms. $A_1$ and $B_1$ are each preferably an optionally substituted alkylene group with 1-3 carbon atoms, and more preferably an alkylene group with 1 or 2 carbon atoms.

In general formula (1-1), $m_1$ and $n_1$ are each independently an integer from 0 to 6. Preferably, $m_1$ and $n_1$ are each an integer from 0 to 3, more preferably an integer from 0 to 2, and particularly preferably 0 or 1.

In general formula (1-1), $a_1$ and $b_1$ are each independently an integer from 0 to 10. Preferably, $a_1$ and $b_1$ are each an integer from 0 to 5, more preferably an integer from 0 to 3, still more preferably an integer from 0 to 2, and particularly preferably 0 or 1.

In general formula (1-1), $R_1$' and $R_1$" are each independently selected from the group consisting of a hydrogen atom, an optionally substituted alkyl group with 1-20 carbon atoms, and an optionally substituted aryl group with 6-20 carbon atoms; Preferably, $R_1$' and $R_1$" are each an integer from 0 to 5, more preferably an integer from 0 to 3, still more preferably an integer from 0 to 2, and particularly preferably 0 or 1.

As is evident from the fact that $R_1$' and $R_1$" in general formula (1-1) can be independently selected from a hydrogen atom, an alkyl group, and an aryl group, the dicarboxylic acid compound herein includes not only dicarboxylic

acid but also a monocarboxylic acid monoester compound and a carboxylic acid diester compound.

Specific examples of the dicarboxylic acid compound represented by general formula (1-1) include 2,2'-([1,1'-binaphthalene]-2,2'-diylbis(oxy))acetoacetic acid (BINOL-DC), 2,2'-bis(ethoxycarboxymethoxy)-1,1'-binaphthyl, 2,2'-bis(2-ethoxycarboxyethoxy)-1,1'-binaphthyl, 2,2'-bis(3-ethoxycarboxypropoxy)-1,1'-binaphthyl, 2,2'-bis(3-ethoxycarboxy-2-methylpropoxy)-1,1'-binaphthyl, 2,2'-bis(4-ethoxycarboxyphenylmethoxy)-1,1'-binaphthyl, 2,2'-biphenyldicarboxylic acid, 2,2'-bis(methoxycarboxymethoxy)-1,1'-binaphthyl, 2,2'-bis(2-methoxycarboxyethoxy)-1,1'-binaphthyl, 2,2'-bis(3-methoxycarboxypropoxy)-1,1'-binaphthyl, 2,2'-bis(3-methoxycarboxy-2-methylpropoxy)-1,1'-binaphthyl, 2,2'-bis(4-methoxycarboxyphenylmethoxy)-1,1'-binaphthyl, 2,2'-biphenyldicarboxylic acid dimethyl, 2,2'-biphenyldicarboxylic acid diethyl, and 2,2'-biphenyldicarboxylic acid diphenyl.

In addition, specific examples of the dicarboxylic acid compound represented by general formula (1-1), which has a fluorene group, include 9,9-bis(carboxynaphthyl)fluorene, 9,9-bis(carboxy-alkylphenyl)fluorene, and 9,9 bis(carboxyphenyl)fluorene.

Examples include:

9,9-bis(carboxynaphthyl)fluorene, such as 9,9-bis(6-carboxy-2-naphthyl)fluorene and 9,9-bis(5-carboxy-1-naphthyl)fluorene;

9,9-bis(carboxy-alkylphenyl)fluorene including 9,9-bis(carboxy-(mono or di)C1-4 alkylphenyl)fluorene, such as 9,9-bis(4-carboxy-3-methylphenyl)fluorene and 9,9-bis(4-carboxy-3,5-dimethylphenyl)fluorene;

9,9-bis(carboxyphenyl)fluorene, such as 9,9-bis(4-carboxyphenyl)fluorene; and

9,9-bis(carboxy-arylphenyl)fluorene including 9,9-bis(carboxy-C6-10 arylphenyl)fluorene, such as 9,9-bis(4-carboxy-3-phenylphenyl)fluorene.

Among the specific examples of the dicarboxylic acid compound represented by formula (1-1) above, BINOL-DC, 2,2'-bis(ethoxycarboxymethoxy)-1,1'-binaphthyl, 2,2'-bis(methoxycarboxymethoxy)-1,1'-binaphthyl, etc. are preferred.

Note that a mixed ester may also be used as the dicarboxylic acid compound.

Each substituent in general formula (1-2) may be synonymous with the corresponding substituent in general formula (1-1) above.

Specifically, $R_{a2}$ and $R_{b2}$ in general formula (1-2) may be synonymous with $R_{a1}$ and $R_{b1}$ in formula (1-1), and $R_{a2}$ and $R_{b2}$ in general formula (1-2) may each independently be selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h2}$.

In addition, $R_{h2}$ in general formula (1-2) may be synonymous with $R_{h1}$ in formula (1-1), and may represent an optionally substituted aryl group with 6-20 carbon atoms or an optionally substituted heteroaryl group with 6-20 carbon atoms containing one or more heteroatoms selected from O, N, and S.

$R_{a2}$ and $R_{b2}$ in general formula (1-2) are each preferably a hydrogen atom, an optionally substituted aryl group with 6-20 carbon atoms, or an optionally substituted heteroaryl group with 6-20 carbon atoms containing one or more heteroatoms selected from O, N, and S, more preferably a hydrogen atom or an optionally substituted aryl group with 6-20 carbon atoms, and still more preferably a hydrogen atom or an optionally substituted aryl group with 6-12 carbon atoms.

$X_2$ in general formula (1-2) may be synonymous with $X_1$ in general formula (1-1), and $X_2$ may represent a single bond or an optionally substituted fluorene group, preferably a single bond.

$A_2$ and $B_2$ in general formula (1-2) may be synonymous with $A_1$ and $B_1$ in general formula (1-1), respectively, and $A_2$ and $B_2$ may each independently represent an optionally substituted alkylene group with 1-5 carbon atoms. $A_1$ and $B_1$ are each preferably an optionally substituted alkylene group with 1-3 carbon atoms, and more preferably an alkylene group with 1 or 2 carbon atoms.

$m_2$ and $n_2$ in general formula (1-2) may be synonymous with $m_1$ and $n_1$ in general formula (1-1), respectively, and $m_2$ and $n_2$ may each independently represent an integer from 0 to 6. Preferably, $m_1$ and $n_1$ are each an integer from 0 to 3, more preferably an integer from 0 to 2, and particularly preferably 0 or 1.

$a_2$ and $b_2$ in general formula (1-2) may be synonymous with $a_1$ and $b_1$ in general formula (1-1), respectively, and $a_2$ and $b_2$ may each independently represent an integer from 0 to 10. Preferably, $a_1$ and $b_1$ are each an integer from 0 to 5, more preferably an integer from 0 to 3, still more preferably an integer from 0 to 2, and particularly preferably 0 or 1.

$R_2$' and $R_2$' in general formula (1-2) may be synonymous with $R_1$' and $R_1$' in general formula (1-1), respectively, and $R_2$' and $R_2$" may be each independently selected from the group consisting of a hydrogen atom, an optionally

substituted alkyl group with 1-20 carbon atoms, and an optionally substituted aryl group with 6-20 carbon atoms. Preferably, $R_1$' and $R_1$" are each an integer from 0 to 5, more preferably an integer from 0 to 3, still more preferably an integer from 0 to 2, and particularly preferably 0 or 1.

As is evident from the fact that $R_2$' and $R_2$" in general formula (1-2) can be independently selected from a hydrogen atom, an alkyl group, and an aryl group, the dicarboxylic acid compound herein includes not only dicarboxylic acid but also a monocarboxylic acid monoester compound and a carboxylic acid diester compound.

In addition, each substituent in general formula (1-2) may be different from the substituent in general formula (1-1) above.

For example, $R_{a2}$ and $R_{b2}$ in general formula (1-2) may be synonymous with $R_e$ and $R_f$ in general formula (2-2), respectively, and $R_{a2}$ and $R_{b2}$ may be each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms. Preferred choices for $R_{a2}$ and $R_{b2}$ may be identical to those for $R_e$ and $R_f$ in general formula (2-2) described below.

In addition, $X_2$ in general formula (1-2) may be synonymous with $Y_2$ in general formula (2-2), and $X_2$ may be a single bond, an optionally substituted fluorene group, or any of the structural formulae represented by formulae (8)-(15) above. Preferred choices for $X_2$ may be identical to those for $Y_2$ in general formula (2-2) described below.

$A_2$ and $B_2$ in general formula (1-2) may be synonymous with $E_2$ and $F_2$ in general formula (2-2), respectively, and $A_2$ and $B_2$ may each independently represent an optionally substituted alkylene group with 1-5 carbon atoms. Preferred choices for $A_2$ and $B_2$ may be identical to those for $E_2$ and $F_2$ in general formula (2-2) described below.

In addition, $m_2$ and $n_2$ in general formula (1-2) may be synonymous with t and u in general formula (2-2), respectively, and $m_2$ and $n_2$ may each independently represent an integer from 0 to 4. Preferred choices for $m_2$ and $n_2$ may be identical to those for t and u in general formula (2-2) described below.

$a_2$ and $b_2$ in general formula (1-2) may be synonymous with e and f in general formula (2-2), respectively, and $a_2$ and $b_2$ may each independently represent an integer from 0 to 10. Preferred choices for $a_2$ and $b_2$ may be identical to those for e and f in general formula (2-2) described below.

Specific examples of the dicarboxylic acid compound represented by general formula (1-2) include those in which the naphthyl ring of the dicarboxylic acid compound represented by formula (1-1) is replaced by a benzene ring. Examples of the dicarboxylic acid compound represented by general formula (1-2) include 9,9-bis(carboxy C2-6 alkyl)-dipyrenylfluorene, such as 9,9-bis(2-carboxyethyl)-2,7-di(1-pyrenyl)fluorene, 9,9-bis(2-carboxyethyl)-1,8-di(1-pyrenyl)fluorene, 9,9-bis(2-carboxyethyl)-3,6-di(1-pyrenyl)fluorene, 9,9-bis(2-carboxyethyl)-4,5-di(1-pyrenyl)fluorene, 9,9-bis(2-carboxyethyl)-2,7-di(2-pyrenyl)fluorene, and 9,9-bis(2-carboxypropyl)-2,7-di(1-pyrenyl)fluorene.

The dicarboxylic acid compound described above is preferably crystallized from a liquid mixture containing not only the dicarboxylic acid compound but also a dihydroxy compound represented by at least either general formula (2-1) below or general formula (2-2) below.

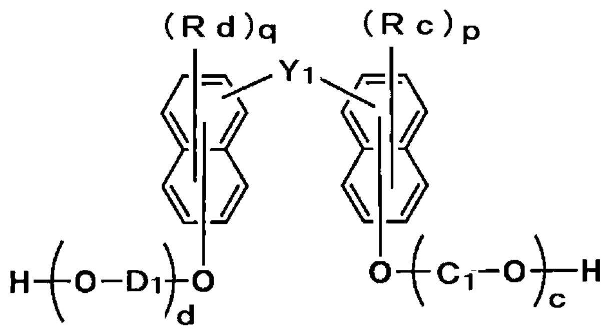

(2 − 1)

In general formula (2-1), $R_c$ and $R_d$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h3}$.

$R_{h3}$ is an optionally substituted aryl group with 6-20 carbon atoms, or an optionally substituted heteroaryl group with 6-20 carbon atoms containing one or more heteroatoms selected from O, N, and S. Preferably $R_{h3}$ is an optionally substituted aryl group with 6-20 carbon atoms, and more preferably an optionally substituted aryl group with 6-12 carbon

atoms.

$R_c$ and $R_d$ in general formula (2-1) are each preferably a hydrogen atom, an optionally substituted aryl group with 6-20 carbon atoms, or an optionally substituted heteroaryl group with 6-20 carbon atoms containing one or more heteroatoms selected from O, N, and S, more preferably a hydrogen atom or an optionally substituted aryl group with 6-20 carbon atoms, and still more preferably a hydrogen atom or an optionally substituted aryl group with 6-12 carbon atoms.

In general formula (2-1), $Y_1$ represents a single bond or an optionally substituted fluorene group. $Y_1$ in general formula (2-1) may be synonymous with $Y_2$ described below. $Y_1$ is preferably a single bond, or an optionally substituted fluorene group with a total of 12-20 carbon atoms.

In general formula (2-1), $C_1$ and $D_1$ are each independently an optionally substituted alkylene group with 1-5 carbon atoms, and preferably an alkylene group with 2 or 3 carbon atoms.

In general formula (2-1), p and q are each independently an integer from 0 to 6, preferably an integer from 0 to 3, and more preferably 0 or 1.

In general formula (2-1), c and d are each independently an integer from 0 to 10, preferably an integer from 1 to 3, and more preferably 1 or 2.

Specific examples of the dihydroxy compound represented by (2-1) include 2,2'-bis(2-hydroxyethoxy)-1,1'-binaphthalene (BNE), and 2,2'-bis(2-hydroxyethoxy)-6,6'-diphenyl-1,1'-binaphthalene (DPBHBNA).

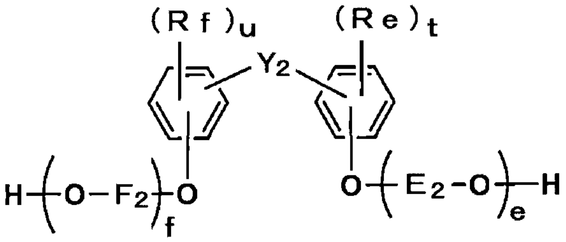

(2−2)

In general formula (2-2), $R_e$ and $R_f$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms.

$R_e$ and $R_f$ are each preferably a hydrogen atom, an optionally substituted aryl group with 6-20 carbon atoms, or an optionally substituted heteroaryl group with 6-20 carbon atoms containing one or more heteroatoms selected from O, N, and S, more preferably a hydrogen atom or an optionally substituted aryl group with 6-20 carbon atoms, and still more preferably a hydrogen atom or an optionally substituted aryl group with 6-12 carbon atoms.

In general formula (2-2), $Y_2$ is a single bond, an optionally substituted fluorene group, or any of the structural formulas represented by formulae (8)-(15) below, and preferably a single bond or a structural formula represented by formula (8) below.

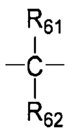

(8)

$-S-$

(9)

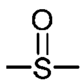

(10)

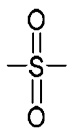

(11)

$-(CH_2)_r-$

(12)

$-O-$

(13)

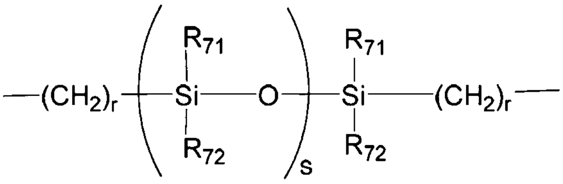

(14)

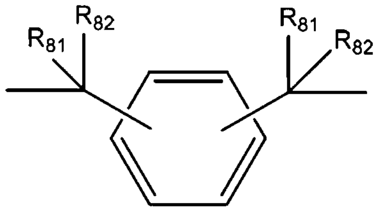

(1 5)

In formulae (8)-(15), $R_{61}$, $R_{62}$, $R_{71}$, $R_{72}$, $R_{81}$, and $R_{82}$ each independently represent a hydrogen atom, a halogen atom, an optionally substituted alkyl group with 1-20 carbon atoms, or an optionally substituted aryl group with 6-30 carbon atoms, or $R_{61}$ and $R_{62}$ or $R_{71}$ and $R_{72}$ bond to each other to form an optionally substituted carbon or heterocyclic ring with 1-20 carbon atoms.

Also in formulae (8)-(15), r and s each independently represent an integer from 0 to 5,000.

In general formula (2-2), $E_2$ and $F_2$ are each independently an optionally substituted alkylene group with 1-5 carbon atoms, and preferably an alkylene group with 2 or 3 carbon atoms.

In general formula (2-2), t and u each independently represent an integer from 0 to 4, and preferably 0 or 1.

In general formula (2-2), e and f are each independently an integer from 0 to 10, preferably an integer from 0 to 5, more preferably an integer from 0 to 2, and, for example, 0 or 1.

Specific examples of the dihydroxy compound represented by general formula (2-2) include BCFL (9,9-bis(4-hydroxy-3-methylphenyl)fluorene), BPEF (9,9-bis(4-(2-hydroxyethoxy)phenyl)fluorene), BPPEF (9,9-bis(4-(2-hydroxyethoxy)-3-phenylphenyl)fluorene), BNEF (9,9-bis[6-(2-hydroxyethoxy)naphthalene-2-yl]fluorene), bisphenol A, bisphenol AP, bisphenol AF, bisphenol B, bisphenol BP, bisphenol C, bis(4-hydroxyphenyl)-2,2-dichloroethylene, bisphenol E, bisphenol F, bisphenol G, bisphenol M (BPM), bisphenol S, bisphenol P, bisphenol PH, bisphenol TMC, bisphenol P-AP (4,4'-(1-phenylethylidene)bisphenol), bisphenol P-CDE (4,4'-cyclododecylidenebisphenol), bisphenol P-HTG (4,4'-(3,3,5-trimethylcyclohexylidene)bisphenol), bisphenol P-MIBK (4,4'-(1,3-dimethylbutylidene)bisphenol), bisphenol PEO-FL (bisphenoxyethanol fluorene), bisphenol P-3MZ (4-[1-(4-hydroxyphenyl)-3-methylcyclohexyl]phenol), bisphenol OC-FL (4,4'-[1-[4-[1-(4-hydroxyphenyl)-1-methylethyl]phenyl]ethylidene]bisphenol), bisphenol Z, BP-2EO (2,2'-[[1,1'-biphenyl]-4,4'-diylbis(oxy)bisethanol), S-BOC (4,4'-(1-methylethylidene)bis(2-methylphenol), and TrisP-HAP (4,4',4"-ethylidene trisphenol). Among these, BPEF, BNEF, BPM, and BCFL are preferred as the dihydroxy compound represented by general formula (2-2).

1-2. Steps in method for producing dicarboxylic acid compound

A method for producing a dicarboxylic acid compound of the present invention comprises a crystallization step in which the aforementioned dicarboxylic acid compound is crystallized. According to the production method adopting such a crystallization step, for example, a dicarboxylic acid compound, etc. can be obtained as a desired resin-derived monomer compound from a waste resin composition that may contain impurities. A dihydroxy compound can also be produced. In other words, according to the production method of the present invention, a high purity dicarboxylic acid compound, dihydroxy compound, etc. can be produced from a waste resin composition. This also enables the production of a high-quality resin by producing a resin (recycled resin) using the obtained dicarboxylic acid compound, dihydroxy compound, etc., thereby allowing for the preferable recycling of a waste resin composition.

(Decomposition step)

The method for producing a dicarboxylic acid compound of the present invention may comprise a decomposition step to produce, for example, a dicarboxylic acid compound from a waste resin composition. In the decomposition step, a polyester carbonate resin, a polyester resin, etc., which has a structural unit derived from a dicarboxylic acid compound represented by at least general formula (1-1') below or general formula (1-2') below, is decomposed (depolymerized).

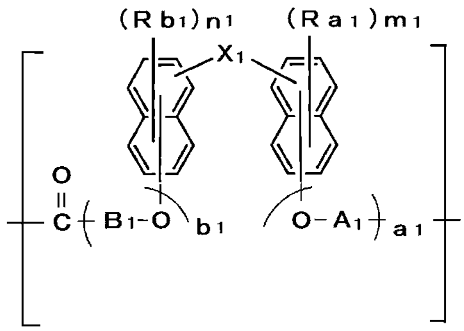

(1－1’)

Each symbol in general formula (1-1’) is synonymous with the one in general formula (1-1) above.

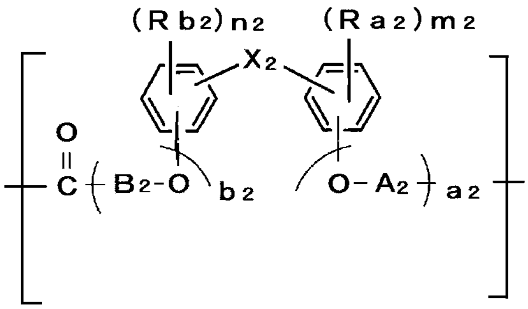

(1－2’)

Each symbol in general formula (1-2’) is synonymous with the one in general formula (1-2) above.

In the decomposition step, the dicarboxylic acid compound represented by general formula (1-1) or general formula (1-2) described above can be obtained efficiently. A waste resin composition can be utilized as the resin or resin composition that undergoes the decomposition step.

Herein, the waste resin composition includes: a resin composition that was once made into a product, but is no longer used for that product application; a resin composition that is discarded regardless of whether it has been used or not; and the like.

In the decomposition step, at least either the polyester carbonate resin or the polyester resin is preferably dissolved in a first solvent and treated with an alkaline solution to obtain a reaction solution containing the first solvent and the dicarboxylic acid compound represented by formula (1-1) or formula (1-2) above. In the subsequent crystallization step described above, the dicarboxylic acid compound is preferably crystallized from a liquid mixture subjected to crystallization which is obtained by adding a second solvent to the reaction solution.

(Alkaline solution)

The alkaline solution contains, for example, a first solvent and water. The alkaline solution may further contain a metal oxide or the like.

The resin to be decomposed in the decomposition step may further contain a structural unit other than those derived from the dicarboxylic acid compound and dihydroxy compound. This structural unit is not particularly limited, and examples thereof include structural units derived from olefins and structural units derived from esters. The resin may include the aforementioned other structural units alone or in combination of two or more.

The above-mentioned resin may be contained in the resin alone or in combination of two or more.

While the weight average molecular weight (Mw) of the resin to be decomposed in the decomposition step is not particularly limited, it is preferably 10,000-70,000 and more preferably 15,000-50,000. The weight average molecular weight (Mw) of the above resin is preferably 10,000 or more so that it can retain an appropriate strength as a molded body, such as a resin for optical lenses. On the other hand, the weight average molecular weight (Mw) of the resin is preferably 70,000 or less so that it can retain an appropriate fluidity during resin molding and its moldability can be improved.

In the resin to be decomposed in the decomposition step, the content of a resin derived from a waste resin composition described below is preferably 80% by mass or more and more preferably 80-99% by mass. The content of the resin derived from the waste resin composition is preferably 80% by mass or more for higher efficiency.

Organic impurities

The resin composition to be decomposed in the decomposition step may contain organic impurities, such as impurity resins and impurity compounds.

Among them, the impurity resin is preferably a polyolefin resin or a cyclic polyolefin, and more preferably a cyclic polyolefin from the viewpoint that there is a significant difference in chemical properties (significant difference in ease of depolymerization in an aqueous alkaline solution) from the polyester carbonate resin, etc.

First solvent

The first solvent has the functions of promoting the depolymerization that leads to decomposition in the decomposition step, dissolving the dihydroxy compound obtained by the depolymerization, and the like. Examples of the first solvent include, but are not particularly limited to, aliphatic hydrocarbon-based solvents and aromatic hydrocarbon-based solvents.

Examples of the aliphatic hydrocarbon-based solvent include, but are not particularly limited to, pentane, hexane, heptane, octane, nonane, decane, cyclohexane, and cyclodecane.

Examples of the aromatic hydrocarbon-based solvent include, but are not particularly limited to, toluene, xylene, and mesitylene.

Among them, the first solvent is preferably be an aromatic hydrocarbon-based solvent, and more preferably toluene, xylene or the like. Note that the above reaction solvents may be used alone or in combination of two or more.

While the amount of the first solvent used is not particularly limited, it is preferably 30-2,000 parts by mass, more preferably 40-1,500 parts by mass, and still more preferably 100-1,000 parts by mass, relative to 100 parts by mass of the resin composition to be decomposed in the decomposition step. The amount of the first solvent used is preferably 30 parts by mass or more so that the organic components in the waste resin composition are fully dissolved in the reaction solvent, resulting in high reaction efficiency. On the other hand, the amount of the first solvent used is preferably 2,000 parts by mass or less so that the reaction time is shortened.

Water

Water has the functions of promoting the depolymerization of the resin composition, and the like.

While the amount of water used is not particularly limited, it is preferably 10-2,000 parts by mass, more preferably 20-500 parts by mass, still more preferably 30-300 parts by mass, and particularly preferably 40-100 parts by mass, relative to 100 parts by mass of the resin composition.

Metal oxide

The metal oxide has the functions of adjusting the reaction solution in the decomposition step to alkalinity, thereby promoting depolymerization, and the like.

Examples of the metal oxide include, but are not particularly limited to: alkali metals, such as sodium hydroxide, potassium hydroxide, and rubidium hydroxide; and alkaline earth metals, such as calcium hydroxide and barium hydroxide. Among them, the metal oxide is preferably an alkali metal, more preferably sodium hydroxide or potassium hydroxide, and still more preferably potassium hydroxide. Note that these metal oxides may be used alone or in combination of two or more.

While the amount of the metal oxide used is not particularly limited, it is preferably 1.5-10 mol, more preferably 2-8 mol, and still more preferably 2-4 mol per mol of ester bond in the polyester carbonate resin, etc. The amount of the metal oxide used is preferably 1.5 mol or more so that the depolymerization can be carried out sufficiently. On the other hand, the amount of the metal oxide used is preferably 10 mol or less so that the production cost can be reduced.

The concentration of the metal oxide in the aqueous alkaline solution is preferably 10-60% by mass, more preferably 15-55% by mass, and still more preferably 20-50% by mass, relative to the total mass of the alkaline solution. The concentration of the metal oxide is preferably 10% by mass or more so that the reaction rate of depolymerization can be increased. On the other hand, the concentration of the metal oxide is preferably 60% by mass or less so that the aqueous alkaline solution does not turn into a slurry and the reaction proceeds more easily.

(Treatment)

By treating a resin such as a polyester carbonate resin with an alkaline solution as described above, a reaction solution, which contains a mixture containing the first solvent, the dicarboxylic acid compound, and, if any, other dihydroxy compound, etc., can be obtained.

The organic impurities that may be contained in the resin composition have different chemical properties in depolymerization from the resins described above. Therefore, the organic impurity will not react, depolymerize earlier than the above-mentioned resin, depolymerize later than the aforementioned resin, etc. For example, if the organic impurities are polyolefin resins, cyclic polyolefins, or the like, these compounds usually do not react under basic conditions, and only the aforementioned resins will be depolymerized. Therefore, they can be easily removed. Even if the organic impurities are depolymerized, the depolymerized products of the organic impurities can be easily removed because the depolymerized products usually have polarities different from that of the dihydroxy compound.
While the treatment temperature (depolymerization reaction temperature) in the decomposition step is not particularly limited, it is preferably 120°C or lower, more preferably 100°C or lower, and still more preferably 30-90°C. The treatment temperature is preferably 120°C or lower so that side reactions can be prevented.
Note that the solution obtained after the treatment (depolymerization) in the decomposition step can be appropriately purified by washing, extraction, or the like, to give a reaction solution for further crystallization step. Note that the solution obtained after the treatment (depolymerization) usually contains an organic phase derived from the first solvent and an aqueous phase derived from water. In this case, the dicarboxylic acid compound, etc. obtained by the depolymerization can be contained in the organic phase. For this reason, the aqueous phase derived from water is preferably removed from the solution obtained after the treatment (depolymerization), by liquid-liquid extraction or the like.
In addition, the organic impurities and depolymerized products thereof contained in the obtained reaction solution, when they have physical properties different from those of the dicarboxylic acid compound, can be appropriately purified and removed at this stage. In this case, the reaction solution and the solution subjected to crystallization described below contain no or little organic impurities and depolymerized products thereof.

(Reaction solution)

The reaction solution preferably contains the dicarboxylic acid compound, etc. and the first solvent. The reaction solution may further contain organic impurities, depolymerized products thereof, and the like.

First solvent

As the first solvent, those described above can be used. As for the content of the first solvent, note that the amount of the first solvent can be adjusted by distilling the solvent off or the like in the treatment after depolymerization. By doing this, the content of the first solvent in the solution subjected to crystallization described below can be adjusted.

Organic impurities and depolymerized products thereof

The reaction solution may contain organic impurities and depolymerized products thereof.
Since organic impurities usually differ greatly from the dicarboxylic acid compound in terms of molecular weight and polarity, they can easily be removed in the washing step described below.
Examples of the depolymerized products of the organic impurities include, but are not particularly limited to, monomers and oligomers obtained by the depolymerization of the organic impurities. For example, when a polyester resin is contained as an organic impurity, an alcohol and a carboxylic acid can be obtained by the depolymerization. Among these depolymerized products, compounds, such as alcohols, differ greatly from carboxylic acids, such as the dicarboxylic acid compound, in terms of polarity, and thus they can be easily removed in the washing step described below.

(Solution forming step)

A method for producing a dicarboxylic acid compound of the present invention may comprise a solution forming step for preparing a liquid mixture which contains the dicarboxylic acid compound and which is suitable for crystallization. In the solution forming step, the dicarboxylic acid compound may be dissolved in a polar solvent to obtain a liquid mixture (first solution forming step) or the dicarboxylic acid compound may be dissolved in an organic solvent to obtain a liquid mixture (second solution forming step).
Examples of the polar solvent include: ketone solvents, such as methyl ethyl ketone (MEK), methyl isobutyl ketone (MIBK), diisobutyl ketone (DIBK), acetone, and cyclohexanone; ether solvents, such as tetrahydrofuran, 1,3-dioxolane, and 1,4-dioxane; and alcohol solvents, such as methanol, ethanol, propanol, isopropyl alcohol, butanol, and isobutyl alcohol. Among the above specific examples, the solvent in the first solution forming step is preferably a ketone solvent, and more preferably methyl ethyl ketone (MEK). Other than MEK, a ketone solvent with 5 or less or 4 or less carbon atoms, a ketone solvent with 3 or more carbon atoms, or the like can be suitably used. Note that these solvents may be used alone or in combination of two or more.
In a step involving acid-base extraction of the dicarboxylic acid compound from an aqueous phase containing the

dicarboxylic acid compound, it is preferable that the aqueous phase is first washed with a relatively-low-polarity solvent, such as an organic solvent described below, then the aqueous phase is acidified, and the dicarboxylic acid compound is extracted from the aqueous phase using a solvent, such as the aforementioned ketone solvent, butanol, isobutyl alcohol, or other alcohol primarily a monohydric alcohol with around 4-6 carbon atoms. For example, when the solvent is selected as described above, the dicarboxylic acid compound contained in the acidic aqueous phase as a free acid can undergo efficient acid-base extraction using the aforementioned high-polarity solvent, such as a ketone.

Examples of the organic solvent used in the second solution forming step include, but are not particularly limited to, low-polarity solvents, including: aliphatic hydrocarbon-based solvents, such as pentane, hexane, heptane, octane, nonane, decane, cyclohexane, and cyclodecane; halogenated aliphatic hydrocarbon solvents, such as dichloromethane and chloroform; and aromatic hydrocarbon-based solvents, such as toluene, xylene, and mesitylene.

A mixed solvent of such a low-polarity organic solvent (hereinafter referred to as a low-polarity solvent) and an organic solvent with a higher polarity (hereinafter referred to as a high-polarity solvent) may also be used. For example, a mixed solvent of a low-polarity solvent selected from the aforementioned choices and a high-polarity solvent mentioned above as the polar solvent, such as a ketone solvent, where the mixed solvent contains a 10-90% by mass low-polarity solvent, a 30-70% by mass low-polarity solvent, or a 40-60% by mass low-polarity solvent, relative to the total mass, with the remaining component being a high-polarity solvent, can be used.

In the solution forming step, only the dicarboxylic acid compound can be selectively crystallized from the organic layer, which is obtained by washing a liquid mixture of the dicarboxylic acid compound and the dihydroxy compound dissolved in a polar solvent with an acidic aqueous solution in the washing step described below.

Alternatively, in the solution forming step, a co-crystal of the dicarboxylic acid compound and the dihydroxy compound can be crystallized from a liquid mixture, which is obtained by dissolving the dicarboxylic acid compound and the dihydroxy compound in an organic solvent.

The amount of the solvent used in the solution forming step is preferably 0.01-100 g/g, more preferably 0.1-50 g/g, still more preferably 1.0-20 g/g, and particularly preferably 2.0-10 g/g, relative to the total amount of the dicarboxylic acid compound or to the total amount of the dicarboxylic acid compound and the dihydroxy compound.

The liquid mixture prepared in the solution forming step may contain a dicarboxylic acid compound alone, or a dicarboxylic acid compound and a dihydroxy compound, as the compound to be crystallized. The ratio (mole ratio) of the dicarboxylic acid compound to the dihydroxy compound in the liquid mixture is, for example, 100:0-10:90, preferably 80:20-15:85 or 70:30-20:80, more preferably 60:40-35:65 or 55:45-40:60, and still more preferably 50:50-40:60.

(Washing step)

The method for producing a dicarboxylic acid compound of the present invention may comprise a washing step in which the liquid mixture containing the dicarboxylic acid compound is washed. In the washing step, the liquid mixture containing the dicarboxylic acid compound may be washed with an acidic aqueous solution (first washing step) or the liquid mixture may be washed with an organic solvent (second washing step).

An aqueous solution of an inorganic acid such as hydrochloric acid or nitric acid can be used as the acidic aqueous solution in the washing step, and the concentration of the acid in such an aqueous inorganic acid solution is, for example, 0.1 M-10 M, preferably 0.3 M-5 M, and more preferably 0.5 M-3 M.

In addition, an aqueous solution of an organic acid such as toluene sulfonic acid, oxalic acid, maleic acid, or methanesulfonic acid may be used as the acidic aqueous solution in the washing step. Thus, if an acid having a higher acidity than that of the target compound, dicarboxylic acid compound, such as BINOL-DC, is used as an acidic aqueous solution in the washing step, the salt of the dicarboxylic acid compound can be converted to a carboxylic acid, thereby enabling efficient crystallization.

The organic solvent used in the washing step may be the same as the one used in the solution forming step, for example, an aliphatic hydrocarbon-based solvent, such as pentane, hexane, heptane, octane, nonane, decane, cyclohexane, or cyclodecane, or an aromatic hydrocarbon-based solvent, such as toluene, xylene, or mesitylene.

(Crystallization step)

The method for producing a dicarboxylic acid compound of the present invention comprises a crystallization step in which at least the dicarboxylic acid compound is crystallized. In the crystallization step, a pure dicarboxylic acid compound can be efficiently crystallized from the liquid mixture. Thus, the dicarboxylic acid compound obtained in the crystallization step is typically in a crystalline form.

The liquid mixture that undergoes the crystallization step is obtained, for example, by adding a second solvent to the reaction solution obtained in the decomposition step described above.

Specifically, the mixed solution subjected to crystallization preferably contains the dicarboxylic acid compound, the dihydroxy compound, and the first solvent contained in the reaction solution described above, and the second solvent.

Additionally, the mixed solution may further contain a seed crystal, organic impurities, and depolymerized products thereof.

The content of the dicarboxylic acid compound in the mixed solution subjected to crystallization is preferably 1-35% by mass, more preferably 5-30% by mass, still more preferably 5-25% by mass, and particularly preferably 10-25% by mass, relative to the total mass of the mixed solution. When two or more types of dicarboxylic acid compounds are contained, the total content thereof is preferably in the aforementioned ranges.

First solvent

The first solvent may be any of those mentioned above. The first solvent typically dissolves the dicarboxylic acid compound and the dihydroxy compound.

In this case, the solubility of the dicarboxylic acid compound in the first solvent at 25°C is preferably 0.01 g/10 mL or more each, more preferably 0.02 g/10 mL or more each, and still more preferably 0.05 g/10 mL or more. Note that, while the upper limit of the solubility of the dicarboxylic acid compound in the first solvent at 25°C is not particularly limited, it is 3 g/10 mL or less each, preferably 2 g/10 mL or less each, and more preferably 1 g/10 mL or less each from the viewpoint of performing suitable crystallization.

The content of the first solvent in the mixed solution subjected to crystallization is preferably 0.1-10 g/g, more preferably 0.5-7.5 g/g, and still more preferably 0.75-5 g/g, relative to the total amount of the dicarboxylic acid compound. The content of the first solvent is preferably in the above range so that a highly pure dicarboxylic acid compound can be obtained by crystallization. Note that the content of the first solvent in the solution subjected to crystallization can be adjusted by distilling the solvent off or the like after depolymerization. When the dihydroxy compound is also to be crystallized, the content of the first solvent in the mixed solution is preferably 0.1-10 g/g, more preferably 0.5-7.5 g/g, and still more preferably 0.75-5 g/g, relative to the total amount of the mixture of the dicarboxylic acid compound and the dihydroxy compound to be crystallized.

Second solvent

The difference between the solubility of the dicarboxylic acid compound in the second solvent at 25°C and the solubility of the dicarboxylic acid compound in the first solvent at 25°C (the solubility difference at 25°C) is preferably 0.1 g/10 mL or more, more preferably 0.3 g/10 mL or more, and still more preferably 0.4-10 g/10 mL.

In addition, the difference between the solubility of the dicarboxylic acid compound in the second solvent at 70°C and the solubility of the dicarboxylic acid compound in the first solvent at 70°C (the solubility difference at 70°C) is preferably 0.1 g/10 mL or more, more preferably 0.3 g/10 mL or more, and particularly preferably 0.4-10 g/10 mL.

As the second solvent, the one used in the aforementioned solution forming step may be used, and examples thereof include: ketone solvents, such as acetone, methyl ethyl ketone (MEK), methyl isobutyl ketone (MIBK), diisobutyl ketone (DIBK), and cyclohexanone; ether solvents, such as tetrahydrofuran, 1,3-dioxolane, and 1,4-dioxane; and alcohol solvents, such as ethanol, propanol, isopropyl alcohol, butanol, and isobutyl alcohol. Among them, the second solvent is preferably a ketone solvent, and more preferably methyl ethyl ketone (MEK). Note that these solvents may be used alone or in combination of two or more.

Note that the type of the dihydroxy compound to be crystallized together with the dicarboxylic acid compound can be determined by selecting the second solvent. For example, when the mixture of the dihydroxy compound contains 2,2'-bis(2-hydroxyethoxy)-6,6'-diphenyl-1,1'-binaphthalene (DP), 2,2'-bis(2-hydroxyethoxy)-1,1'-binaphthalene (BNE), and 9,9-bis[4-(2-hydroxyethoxy)-3-phenylphenyl]fluorene (BPPEF), DP has a solubility of 0.3 g/10 mL, BNE has a solubility of 0.75 g/10 mL, and BPPEF has a solubility of 4.5 g/10 mL, with reference to the solubility in methyl ethyl ketone (MEK) at 25° C. Accordingly, when MEK is used as the second solvent, the first hydroxy compound is DP, and other dihydroxy compounds are BNE and BPPEF, with the solubility difference at 25°C being 0.45 g/10 mL.

The content of the second solvent in the solution subjected to crystallization is preferably 1.0-6.0 g/g, more preferably 0.3-5.0 g/g, and still more preferably 0.75-2 g/g, relative to the total amount of the dicarboxylic acid compound or to the total amount of the mixture of the dicarboxylic acid compound and the dihydroxy compound. The content of the second solvent in the above range is preferable because a highly pure dicarboxylic acid compound can be obtained by crystallization.

The total content of the first solvent and the second solvent in the mixed solution subjected to crystallization is preferably 1-10 g/g and more preferably 1.5-8.0 g/g, relative to the total amount of the dicarboxylic acid compound or to the total amount of the mixture of the dicarboxylic acid compound and the dihydroxy compound. The total content of the first solvent and the second solvent is preferably 1 g/g or more, for example, 2 g/g or more so that the purity of the dicarboxylic acid compound can be high. On the other hand, the total content of the first solvent and the second solvent is preferably 10 g/g or less so that the yield can be higher.

In one embodiment, the total content of the first solvent and the second solvent in the mixed solution subjected to

crystallization is preferably 2 g/g or more and more preferably 4 g/g or more, relative to the total amount of the dicarboxylic acid compound or to the total amount of the mixture of the dicarboxylic acid compound and the dihydroxy compound. The upper limit of the total content of the first solvent and the second solvent is preferably 10 g/g or less. The total content of the first solvent and the second solvent is preferably 2 g/g or more so that the purity of the dicarboxylic acid compound can be high.

The ratio of the content of the first solvent (g/g) to the content of the second solvent (g/g) (first solvent/second solvent) in the mixed solution subjected to crystallization is preferably 0.8-10, more preferably 0.8-7, still more preferably 0.9-4, and particularly preferably 1-3. The ratio (first solvent/second solvent) in the above-mentioned range is preferable because the purity of the dicarboxylic acid compound can be high.

In the crystallization process, a crystal of only the dicarboxylic acid compound may be crystallized from a liquid mixture containing at least the dicarboxylic acid compound, or a co-crystal of the dicarboxylic acid compound and the dihydroxy may be crystallized from the liquid mixture.

Specifically, the (co-)crystal obtained in the crystallization step may contain only the dicarboxylic acid compound, or contain the dicarboxylic acid compound and the dihydroxy compound. The ratio (mole ratio) of the dicarboxylic acid compound to the dihydroxy compound in the (co-)crystal is, for example, 100:0-10:90, preferably 90:10-20:80 or 80:20-30:70, more preferably 75:25-40:60 or 70:30-45:55, and still more preferably 65:35-50:50.

In addition, in the crystallization step, a seed crystal of the dicarboxylic acid compound or the dihydroxy compound may be added to the liquid mixture. Specifically, the dicarboxylic acid compound may be crystallized from the liquid mixture by adding a seed crystal of only the dicarboxylic acid compound in the crystallization step. Alternatively, when the liquid mixture contains, among the dicarboxylic acid compounds and dihydroxy compounds represented by the above general formulae, those that have similar molecular structures to each other, it is also possible to add a seed crystal of either the dicarboxylic acid compound or the dihydroxy compound to the liquid mixture to crystallize a co-crystal of the dicarboxylic acid compound and the dihydroxy compound.

The form of the seed crystal is not particularly limited, but it is preferably a crystalline solvate. In this case, the compound that forms the crystalline solvate is not particularly limited, but it is preferably a dicarboxylic acid compound represented by general formula (1-1) or (1-2) or a dihydroxy compound represented by general formula (2-1) or (2-2), and more preferably a dicarboxylic acid compound. The solvent forming the solvate is not particularly limited, but it is more preferably methanol, toluene, or methyl ethyl ketone. Furthermore, the content of the solvent in the crystalline solvate is preferably 0.3 to 1.5 mol per mol of the compound that forms the crystalline solvate. By using the seed crystal in the form of a crystalline solvate, a highly pure dihydroxy compound can be produced. Note that, the "crystalline solvate" as used herein refers to a crystal form in which the solvent is contained inside the crystal lattice of the seed crystal.

The ratio of the modal diameter to the median diameter (modal diameter/median diameter) of the seed crystal is preferably 2.0 or less, and more preferably 1.0-1.6. The above ratio (modal diameter/median diameter) is preferably 2.0 or less so that the resulting dicarboxylic acid compound can be a mass crystal that is highly pure and easy to handle. Note that, the "modal diameter" as used herein refers to the most frequently occurring diameter with the highest frequency value, and is determined by measuring particle size using a laser diffraction method. In addition, the "median diameter" refers to the particle diameter corresponding to the cumulative frequency of 50% in a cumulative particle size distribution, and is determined by measuring particle size using a laser diffraction method.

The aspect ratio of the seed crystal is preferably 1-8, and more preferably 1-3. Note that, the "aspect ratio" as used herein means the ratio (L/W) of the maximum crystal length L to the width W of the crystal. In this context, the term "maximum crystal length L" refers to a crystal length that is taken as the longest crystal length in a crystal photograph taken with an optical microscope. In addition, the term "width W" refers to a length that makes an angle of 90 degrees with respect to the maximum crystal length and is the longest. Note that, the maximum crystal length L and width W are each an average value calculated by measuring at least 30 crystals randomly selected in the optical micrograph.

The content of the seed crystal is preferably 0.001-1% by mass and more preferably 0.01-1% by mass, relative to the total amount of the mixture of the dicarboxylic acid compound described above or to the total amount of the dicarboxylic acid compound and the dihydroxy compound described above.

Moreover, the content of the seed crystal is preferably 0.001-1% by mass, more preferably 0.005-0.5% by mass, and still more preferably 0.01-0.1% by mass, relative to the total mass of the liquid mixture as the solution subjected to crystallization.

Preferably, in the crystallization step, the dicarboxylic acid compound and other components, such as a dihydroxy compound, are typically heated to be dissolved and then cooled to crystallize.

While the heating temperature before crystallization varies depending on the solvent used, it is preferably 50-90°C, more preferably 60-85°C, and still more preferably 65-80°C.

While the crystallization temperature, i.e., the temperature at which crystals are generated, is not particularly limited, it is preferably -10-60°C, more preferably 0-50°C, and still more preferably 10-40°C.

While the crystallization time is not particularly limited, it is preferably from 5 minutes to 5 hours, more preferably from 10 minutes to 3 hours, and still more preferably from 30 minutes to 2 hours.

(Step of treating waste resin composition)

The method for producing a dicarboxylic acid compound of the present invention may comprise, for example, a step of treating a waste resin composition. The step of treating a waste resin composition is usually performed prior to the decomposition step described above to provide a resin composition containing a polyester carbonate resin, a polyester resin, etc., that are targeted in the decomposition step.

In one embodiment, the step of treating a waste resin composition includes comminuting a waste resin composition feedstock to prepare a waste resin composition. Moreover, in one embodiment, the step of treating a waste resin composition includes removing a metal from a waste resin composition feedstock. In the step of treating a waste resin composition, both of the comminution and the metal removal described above may be performed. The order of the comminution and the metal removal in this case is not particularly limited, however, the metal removal is preferably carried out after the comminution so that the metal removal can be efficiently performed.

(Waste resin composition feedstock)

Examples of the waste resin composition feedstock include, but are not particularly limited to, molded articles that were used as parts of products on the market and then collected, defective products generated during the molding process, molded products (sprues, runners, gates, etc.) concomitantly generated during the molding process, defective products generated during the productization process, and those derived from unused molded articles that are no longer needed. Among them, defective products generated during the molding process, molded products (sprues, runners, gates, etc.) concomitantly generated during the molding process, defective products generated during the productization process, and those originating from unused molded articles that are no longer needed are preferable in terms of less deterioration of the desired synthetic resin; and defective products generated during the molding process, and molded products (sprues, runners, gates, etc.) concomitantly generated during the molding process are more preferable in terms of efficient availability. Note that the above-mentioned molded products and the like may be sorted, and only those containing organic impurities may be used as the waste resin composition feedstock. For example, the sprues can be sorted, and those that can be recycled directly can be removed and used for a product, and those that are left containing organic impurities can be used as a waste resin composition feedstock. Moreover, those from different sources can be mixed and used as a waste resin composition feedstock.

The form of the waste resin composition feedstock is not particularly limited, and examples thereof include: powder, pellets, sheet, film, molded articles, and the like, as well as discarded lens, sheet, and film; defective products and burrs generated during the manufacturing and/or molding process; and manufacturing wastes, solid materials recovered from product wastes using resins, and comminuted products thereof.

The longest diameter of the waste resin composition feedstock is preferably 100 cm or less, more preferably 50 cm or less, and still more preferably 0.5-3 cm. The longest diameter of the waste resin composition feedstock is preferably 100 cm or less so that less energy is required for comminution. Note that, the term "the longest diameter" as used herein refers to the average value of the diameters of 200 randomly selected objects, the diameters each defined as the longest distance between endpoints on the outline of each of the objects.

(Comminution)

The comminution method is not particularly limited, and any method out of compression, impact, shearing, and friction may be adopted.

Examples of comminutors that can be used include: coarse crushers, such as jaw crusher, gyratory crusher, impact crusher, uniaxial crusher, biaxial crusher, etc.; secondary crushers, such as roll crusher, edge runner, disintegrator, semi-autogenous grinding (SAG) mill, crushing roll, hammer mill, roller mill, etc.; and fine comminutors, such as bead mill, ball mill, vibration ball mill, rod mill, jet mill, planetary mill, etc. Among them, it is preferable to use a coarse crusher, and more preferable to use a uniaxial crusher or a biaxial crusher. Specific examples of comminutors include Powerful crushers 35-560, 35-720, 55-770, and 55-1050 (manufactured by TANAKA Co., Ltd.), and Low speed granulators KGA-250 and KGA-350 (manufactured by KAWATA MFG. CO., LTD.). Note that, the above-mentioned comminutors may be used alone or in combination of two or more.

(Metal removal)

The metal removal method is not particularly limited, and examples thereof include a method using magnetic force, a method using wind force, a method using a sieve, a method using specific gravity, and a method using buoyancy. Among them, a method using magnetic force (method using a magnet, method using a metal detector, etc.), a method using specific gravity, and a method using buoyancy (method using salt water) are preferable. Note that these methods

may be used alone or in combination of two or more.

Examples of the metal to be removed include those contained in plastic products, those mixed in during the molding process, and those mixed in during the comminution process.

(Waste resin composition)

A waste resin composition feedstock may be used as is as the waste resin composition, but the waste resin composition is preferably obtained from a waste resin composition feedstock that underwent the comminution, metal removal, etc. However, when comminution and metal removal are not required for the waste resin composition feedstock, it is preferable to use the waste resin composition feedstock as is as a waste resin composition. For example, when the waste resin composition feedstock is uniform in size, the longest diameter of the waste resin composition feedstock is small (e.g., the longest diameter is 5 cm or less), or no metal is contained, it is preferable not to perform the step of preparing the waste resin composition in terms of manufacturing cost.

The waste resin composition contains, for example, a desired synthetic resin and organic impurities. Herein, a "resin" refers to a resin with a weight average molecular weight of 1,000 or more. Herein, a "weight average molecular weight (Mw)" refers to a weight average molecular weight in terms of polystyrene equivalent measured by gel permeation chromatography (GPC).

Desired synthetic resin

The desired synthetic resin may be, for example, a resin having a structural unit derived from a dicarboxylic acid compound represented by the above general formula (1-1) or (1-2), and also a resin having a structural unit derived from a dihydroxy compound represented by the above formula (2-1) or (2-2). Examples of the desired synthetic resin include a polyester carbonate resin, a polyester resin, and a polycarbonate (PC) resin, preferably a polyester carbonate resin and a polyester resin

At least two structural units mentioned above may be contained in the synthetic resin to be treated in the waste resin composition. In this case, the aforementioned structural units may include two or more types of structural units represented by the same general formula, or two or more types of structural units represented by different general formulae. In addition, the above-mentioned structural unit may be combined with a structural unit of another polycarbonate resin, or may be combined with a structural unit of another resin (polyolefin resin, polyester resin) or the like.

While the weight average molecular weight (Mw) of the desired synthetic resin contained in the waste resin composition is not particularly limited, it is preferably 10,000-70,000 and more preferably 15,000-50,000. The weight average molecular weight (Mw) of the desired synthetic resin is preferably 10,000 or more so that it can retain an appropriate strength as a molded body, such as a resin for optical lenses. On the other hand, the weight average molecular weight (Mw) of the desired synthetic resin is preferably 70,000 or less so that it can retain an appropriate fluidity during resin molding and its moldability can be improved.

The content of the desired synthetic resin in the waste resin composition is preferably 80% by mass or more, and more preferably 80-99% by mass, relative to the mass of the waste resin composition. The content of the desired synthetic resin is preferably 80% by mass or more for higher recycling efficiency.

Organic impurities

Examples of the organic impurities include impurity resins and impurity compounds.

Examples of the impurity resin include thermoplastic resins other than the desired synthetic resin.

Examples of the thermoplastic resin include, but are not particularly limited to, polyolefin resins (polyethylene (PE), polypropylene (PP), polyvinyl chloride (PVC), polystyrene (PS), polyvinyl acetate (PVAc), polytetrafluoroethylene (PTFE), etc.), polyurethane (PU) resins, acrylic resins, polyester resins (polyethylene terephthalate (PET), polybutylene terephthalate (PBT)), polyamide (PA) resins, polyacetal resins, cyclic polyolefins (cycloolefin polymers), polyphenylene sulfide (PPS) resins, polysulfone resins, polyether sulfone resins, liquid crystal polymers (LCP), polyether ether ketone (PEEK) resins, polyamideimide (PAI) resins, and copolymers of structural units of these resins (acrylonitrile-styrene copolymer resin (AS resin), acrylonitrile-butylene-styrene copolymer resin (ABS resin), etc.)

The aforementioned structural units may be contained alone or in a combination of two or more in the impurity resin. In addition, the above-mentioned structural units may be combined with a structural unit of another cyclic polyolefin, or may be combined with a structural unit of another resin (polyolefin resin, polyester resin) or the like.

While the weight average molecular weight (Mw) of the impurity resin is not particularly limited, it is preferably 1,000-3,000,000, more preferably 10,000-3,000,000, still more preferably 20,000-1,000,000, and particularly preferably 30,000-500,000. The weight average molecular weight (Mw) of the impurity resin is preferably 1,000 or more for easier separation. On the other hand, the weight average molecular weight (Mw) of the impurity resin is preferably 3,000,000 or

less because when contamination is caused by a trace amount of impurity resin, it is less likely to be a source of gel impurities.

The content of the impurity resin in the waste resin composition is preferably 50% by mass or less, more preferably 0.001-50% by mass, still more preferably 0.01-30% by mass, and particularly preferably 0.1-20% by mass, relative to the total mass of the waste resin composition. The content of the impurity resin is preferably 50% by mass or less for higher efficiency.

Examples of the impurity compound include, but are not particularly limited to, monomers, dimers, copolymers, and oligomers of the above-mentioned impurity resins, aryl alcohols such as phenols, carbonic acid diesters such as diphenyl carbonates, and modified products of the desired synthetic resin. Herein, an "impurity compound" refers to an impurity of an organic compound with a weight average molecular weight of less than 1,000. Therefore, if the weight average molecular weight of the modified product of the recycled resin is 1,000 or more, the modified product of the desired synthetic resin is classified as an impurity resin.

Form of waste resin composition

The form of the waste resin composition is not particularly limited, and examples thereof include powder, granular, and rod forms.

The longest diameter of the waste resin composition is preferably 5 cm or less, more preferably 3 cm or less, still more preferably 0.001-3 cm, particularly preferably 0.01-2 cm, and highly preferably 0.1-1 cm. The longest diameter of the waste resin composition is preferably 5 cm or less from the viewpoint of ease of transportation and progression of depolymerization, and the like.

2. Method for producing recycled resin

The method for producing a recycled resin of the present invention comprises a polymerization step in which a dicarboxylic acid compound represented by formula (1-1) or (1-2) described above is polymerized. According to such a method for producing a recycled resin, for example, a monomer compound derived from a resin composition recycled from the aforementioned waste resin composition can be used to produce a recycled resin such as a polyester resin and a polyester carbonate resin.

(Polymerization step)

The polymerization step adopts any known polymerization technique for polymerizing the dicarboxylic acid compound.

In one embodiment, a polyester resin can be produced as a recycled resin by performing a solution condensation method on a dicarboxylic acid compound obtained by the aforementioned production method and a carbonic acid diester, in the presence of a basic compound catalyst and/or a transesterification catalyst or in the absence of a catalyst.

In terms of adjusting the physical properties of the recycled resin, and the like, a dihydroxy compound can be used along with a dicarboxylic acid compound as monomers produced from the waste resin composition, or only a dihydroxy compound can be used as a monomer.

For example, in one embodiment, a polyester carbonate resin can be produced as a recycled resin by polymerizing a carbonic acid diester with a dicarboxylic acid compound and a dihydroxy compound obtained by the aforementioned production method, in the presence of a basic compound catalyst and/or a transesterification catalyst or in the absence of a catalyst.

Alternatively, a polycarbonate resin can be produced as a recycled resin by polymerizing a carbonic acid diester with a dihydroxy compound, which may be crystallized along with a dicarboxylic acid compound in the aforementioned production method, in the presence of a basic compound catalyst and/or a transesterification catalyst or in the absence of a catalyst.

Examples of the carbonate acid diester include, but are not particularly limited to, diphenyl carbonate, ditolyl carbonate, bis(chlorophenyl) carbonate, m-cresyl carbonate, dimethyl carbonate, diethyl carbonate, dibutyl carbonate, and dicyclohexyl carbonate. Among them, diphenyl carbonate is preferable.

The amount of the carbonic acid diester compound used is preferably 0.97-1.20 mol, more preferably 0.98-1.10 mol, and still more preferably 1.00-1.10 mol per mol of the dicarboxylic acid compound

Examples of the basic compound catalyst include, but are not particularly limited to, alkali metal compounds, alkaline earth metal compounds, and nitrogen-containing compounds.

Examples of the alkali metal compound include, but are not particularly limited to, organic acid salts, inorganic salts, oxides, hydroxides, hydrides, and alkoxides of alkali metals. Specific examples include sodium hydroxide, potassium hydroxide, cesium hydroxide, lithium hydroxide, sodium bicarbonate, sodium carbonate, potassium carbonate,

cesium carbonate, lithium carbonate, sodium acetate, potassium acetate, cesium acetate, lithium acetate, sodium stearate, potassium stearate, cesium stearate, lithium stearate, sodium borohydride, sodium phenylborate, sodium benzoate, potassium benzoate, cesium benzoate, lithium benzoate, disodium hydrogen phosphate, dipotassium hydrogen phosphate, dilithium hydrogen phosphate, disodium phenyl phosphate, and disodium salt, dipotassium salt, dicesium salt or dilithium salt of bisphenol A, and sodium salt, potassium salt, cesium salt or lithium salt of phenol.

Examples of the alkaline earth metal compound include, but are not particularly limited to, organic acid salts, inorganic salts, oxides, hydroxides, hydrides or alkoxides of alkaline earth metal compounds. Specific examples include magnesium hydroxide, calcium hydroxide, strontium hydroxide, barium hydroxide, magnesium bicarbonate, calcium bicarbonate, strontium bicarbonate, barium bicarbonate, magnesium carbonate, calcium carbonate, strontium carbonate, barium carbonate, magnesium acetate, calcium acetate, strontium acetate, barium acetate, magnesium stearate, calcium stearate, calcium benzoate, and magnesium phenyl phosphate.

Examples of the nitrogen-containing compound include, but are not particularly limited to, quaternary ammonium hydroxides, and salts and amines thereof. Specific examples include: quaternary ammonium hydroxides having an alkyl group, an aryl group, etc., such as tetramethylammonium hydroxide, tetraethylammonium hydroxide, tetrapropylammonium hydroxide, tetrabutylammonium hydroxide, and trimethylbenzylammonium hydroxide; tertiary amines, such as triethylamine, dimethylbenzylamine, and triphenylamine; secondary amines, such as diethylamine and dibutylamine; primary amines, such as propylamine and butylamine; imidazoles, such as 2-methylimidazole, 2-phenylimidazole, and benzoimidazole; and ammonia, tetramethylammonium borohydride, tetrabutylammonium borohydride, tetrabutylammonium tetraphenylborate, and tetraphenylammonium tetraphenylborate.

Examples of the transesterification catalyst include salts of zinc, tin, zirconium, and lead. Specific examples include zinc acetate, zinc benzoate, zinc 2-ethylhexanoate, tin(II) chloride, tin(IV) chloride, tin(II) acetate, tin(IV) acetate, dibutyl tin dilaurate, dibutyl tin oxide, dibutyl tin dimethoxide, zirconium acetylacetonate, zirconium oxyacetate, zirconium tetrabutoxide, lead(II) acetate, and lead(IV) acetate.

The basic compound catalyst and the transesterification catalyst mentioned above may be used alone or in combination of two or more.

The amount of the basic compound catalyst or the transesterification catalyst used (the total amount when used in combination) is preferably $1\times10^{-9}$-$1\times10^{-3}$ mol, and more preferably $1\times10^{-7}$-$1\times10^{-4}$ mol per mol of the dicarboxylic acid compound.

The polymerization step, such as melt polycondensation, is desirably performed by melting the monomer components, such as a dicarboxylic acid compound and a carbonic acid diester, in a reaction vessel and then allowing reaction to occur while retaining a monohydroxy compound that to be produced. In order to retain the monohydroxy compound, the pressure can be controlled by closing the reactor, reducing the pressure, increasing the pressure, or the like.

The recycled resin obtained by the method described above can be suitably used for applications to plastic products.

EXAMPLES

Hereinafter, the present invention will be described in detail by way of examples, although the technical scope of the present invention should not be limited thereto. Note that, unless otherwise specified, "%" in the examples represents "% by mass".

(Polymerization example 1 for polyester carbonate)

As feedstocks, 3,290 g (8.79 mol) of 2,2'-bis(2-hydroxyethoxy)-1,1'-binaphthalene (BNE) represented by the following structural formula, 7,000 g (13.0 mol) of 9,9-bis[6-(2-hydroxyethoxy)-2-naphthyl]fluorene (BNEF, also abbreviated as: NOLE) represented by the following structural formula, 6,610 g (16.4 mol) of 2,2'-([1,1'-binaphthalene]-2,2'-diylbis(oxy))acetoacetic acid (BINOL-DC) represented by the following structural formula, and 1,380 g (6.44 mol) of diphenylcarbonate (DPC), 1.30 g of tris(2,4-pentanedionato)aluminum(III) as a catalyst, and 1.63 mL of diethyl (4-methylbenzyl)phosphonate were placed in a 50-liter reactor equipped with a stirrer and a distillation unit, and subjected to nitrogen replacement.

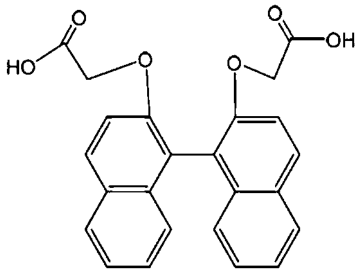

BINOL-DC

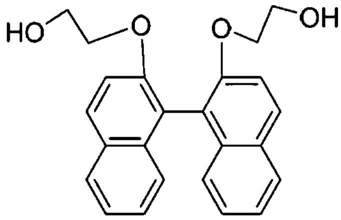

BNE

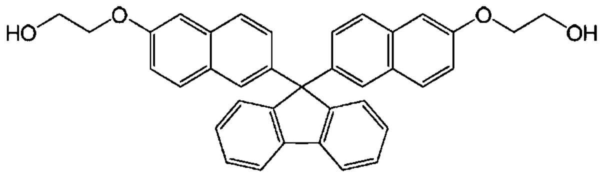

BNEF (NOLE)

In a nitrogen atmosphere of 760 Torr, the reaction system was stirred and heated to 200°C over 20 minutes. After adjusting the degree of depressurization in the reaction system to 300 Torr over 20 minutes, the temperature was increased to 240°C over 40 minutes, and the conditions were maintained at 240°C and 300 Torr for 10 minutes to conduct a transesterification reaction. After returning the pressure to normal with nitrogen gas and collecting water from the trap, the conditions were adjusted again to 240°C and 300 Torr, and the reaction system was held for 10 minutes. Subsequently, after increasing the temperature of the reaction system to 250°C over 70 minutes while adjusting the degree of depressurization to 50 Torr over 50 minutes, the degree of depressurization was further adjusted to 1 Torr or less over 20 minutes to conduct a polymerization reaction with stirring under the conditions of 250°C and 1 Torr or less for 30 minutes. After the reaction was completed, nitrogen was introduced into the reactor to pressurize the reaction system to take out the produced polycarbonate resin while pelletizing, thereby obtaining a polyester carbonate resin.

(Liquid mixture preparation example 1)

100 parts by mass of the polyester carbonate resin obtained in Polymerization example 1, 88 parts by mass of a 48% by mass aqueous sodium hydroxide solution, and 734 parts by mass of toluene as a first solvent were fed into a reactor equipped with a stirrer and a condenser, and reacted while heating and refluxing for 3 hours. In this reaction, the polyester carbonate resin was hydrolyzed to feedstock monomers (dihydroxy compound and dicarboxylic acid compound).

The liquid temperature of the reaction solution was then cooled to 80-85°C, and 155 parts by mass of 35% hydrochloric acid and 30 parts by mass of ion-exchanged water were added. After stirring and allowing the resultant to stand still, the aqueous phase was separated, the organic phase was washed with ion-exchange water, and toluene was partially distilled off. Subsequently, methyl ethyl ketone (MEK) was added as a second solvent in an equal weight to toluene to completely dissolve the dihydroxy compound and the dicarboxylic acid compound while controlling the temperature at 70-75°C, thereby obtaining a liquid mixture (organic).

(Liquid mixture preparation example 2)

100 parts by mass of the polyester carbonate resin obtained in Polymerization example 1, 88 parts by mass of a 48% by mass aqueous sodium hydroxide solution, and 734 parts by mass of toluene as a first solvent were fed into a reactor equipped with a stirrer and a condenser, and reacted while heating and refluxing for 3 hours. In this reaction, the polyester carbonate resin was hydrolyzed to feedstock monomers (dihydroxy compound and dicarboxylic acid compound).

The liquid temperature of the reaction solution was then cooled to 80-85°C, and 188 parts by mass of ion-exchanged water was added. After stirring and allowing the resultant to stand still, the organic phase was separated and removed to obtain a liquid mixture (water soluble). Here, the organic layer had BNE and NOLE dissolved therein, and the aqueous layer had a salt of BINOL-DC dissolved therein.

(Liquid mixture preparation example 3)

3.34 g of BINOL-DC, 1.95 g of BNE, 3.79 g of NOLE, and 40.00 g of toluene (amount added per total gram of dihydroxy compounds and dicarboxylic acid compound (BINOL-DC + BNE + NOLE): 4.00 g) were mixed to prepare Liquid mixture a.
Liquid mixture a was further mixed with 40.00 g of MEK (amount added per total gram of dihydroxy compounds and dicarboxylic acid compound (BINOL-DC + BNE + NOLE): 4.00 g) to prepare Liquid mixture b.
Subsequently, the dihydroxy compounds and the dicarboxylic acid compound were completely dissolved while adjusting the temperature of Liquid mixture b to 70-75°C, thereby obtaining Liquid mixture c (organic).

BINOL-DC/BNE/NOLE (mole ratio) contained in Liquid mixture c was determined to be BINOL-DC/BNE/NOLE (mole ratio) = 43/23/34.

The mole ratio of each of the aforementioned compositions, i.e., the dicarboxylic acid compound and the dihydroxy compounds, was analyzed by high performance liquid chromatography (HPLC). The detailed analysis conditions were as follows, and the mole ratio of each component described below was also analyzed under the same conditions.

Equipment: Agilent Technologies 1260 Infinity
Column: TOSOY TSK-GEL ODS-80-Ts (5 μm, 4.6 mmφ × 250 mm)
Eluent: 0 min acetonitrile/water = 46.3/53.7 (vol%)

: 15 min acetonitrile/water = 46.3/53.7 (vol%)
: 30 min acetonitrile/water = 95/5 (vol%)
: 40 min acetonitrile/water = 95/5 (vol%)

Flow rate: 1 mL/min
Column temperature: 25°C
Measurement wavelength: 254 nm
Injection amount: 5 μm
Sample concentration: 10 mg/10 mL (acetonitrile solvent)
Calculation method: Calculated based on the peak area % (peak intensity) of the chromatograph obtained.

(Example 1)

The temperature of Liquid mixture c obtained in Liquid mixture preparation example 3 above was adjusted to 30°C, and 10 mg of BINOL-DC, which was separately prepared as a seed crystal, was added. The resultant was cooled to around room temperature with air, then cooled further to 10°C in a water bath, and stirred for 1 hour to produce a crystal (crystallization step). The precipitate was filtered, and the resulting precipitate was washed with toluene. The washed precipitate was dried to give 0.69 g of a crystal of the dihydroxy compound and dicarboxylic acid compound. BINOL-DC/BNE/NOLE (mole ratio) in the resulting crystal was determined to be BINOL-DC/BNE/NOLE (mole ratio) = 62/38/0.
The recovery rate was calculated using the following formula and found to be 13%. Note that this recovery rate refers to a value calculated by a conversion assuming that the crystal of the dihydroxy compound and dicarboxylic acid compound was exclusively BINOL-DC and BNE.

$$\text{Recovery rate (\%)} = \frac{\text{Crystal weight (BINOL-DC + BNE)}}{\text{BINOL-DC + BNE weight in solution subjected to crystallization}} \times 100$$
$$= 0.69\ \text{g}/(3.34\ \text{g} + 1.95\ \text{g}) \times 100$$
$$= 13\%$$

(Liquid mixture preparation example 4)

After dissolving 0.167 g of BINOL-DC, 0.097 g of BNE, and 0.189 g of NOLE in 20.00 mL of dichloromethane and 10.00 mL of methanol, 20 mL of a 1M aqueous sodium hydroxide solution was added to the obtained liquid mixture. After stirring and allowing the resultant to stand still, the organic phase was separated to give a liquid mixture (water soluble). Here, the organic layer had BNE and NOLE dissolved therein, and the aqueous layer had a salt of BINOL-DC dissolved therein.

(Example 2)

The liquid mixture (water soluble) containing the salt of BINOL-DC, obtained in Liquid mixture preparation example 4 described above, was washed with dichloromethane. After dichloromethane was removed, dichloromethane was again added to the aqueous phase, and 25 mL of 1M hydrochloric acid was further added to acidify the aqueous phase. After stirring and allowing the resultant to stand still, the aqueous phase was separated and the organic phase was washed with ion-exchange water. Dichloromethane was distilled off from the organic phase to give 0.054 g of a crystal of the dicarboxylic acid compound (BINOL-DC). BINOL-DC/BNE/NOLE (mole ratio) in the resulting crystal was determined to be BINOL-DC/BNE/NOLE (mole ratio) = 100/0/0.

The recovery rate was calculated using the following formula and found to be 32%.

$$\text{Recovery rate (\%)} = \frac{\text{Crystal weight (BINOL-DC)}}{\text{BINOL-DC in solution subjected to crystallization}} \times 100$$
$$= 0.054\ \text{g}/0.167\ \text{g} \times 100$$
$$= 32\%$$

(Example 3)

The liquid mixture (water soluble) containing the salt of BINOL-DC, obtained in Liquid mixture preparation example 4 described above, was washed with dichloromethane. After dichloromethane was removed, 25 mL of 1M hydrochloric acid was added to the liquid mixture to acidify the aqueous phase. Then, MEK (methyl ethyl ketone) was added to the aqueous phase. Here, BINOL-DC can be easily extracted by adding an acid to convert the salt of BINOL-DC into the free acid of BINOL-DC, and then adding MEK, whose polarity is higher than dichloromethane, to the liquid mixture as described above. Then, after stirring and allowing the resultant to stand still, the aqueous phase was separated and the organic phase was washed with ion-exchange water. MEK was distilled off from the organic phase to give 0.141 g of a crystal of the dicarboxylic acid compound. BINOL-DC/BNE/NOLE (mole ratio) in the resulting crystal was determined to be BINOL-DC/BNE/NOLE (mole ratio) = 100/0/0.

The recovery rate was calculated using the following formula and found to be 84%.

$$\text{Recovery rate (\%)} = \frac{\text{Crystal weight (BINOL-DC)}}{\text{BINOL-DC in solution subjected to crystallization}} \times 100$$
$$= 0.141\ \text{g}/0.167\ \text{g} \times 100$$
$$= 84\%$$

According to the above examples, a co-crystal of a dicarboxylic acid compound and a dihydroxy compound can be obtained from a mixture of the dicarboxylic acid compound and the dihydroxy compound through a relatively simple

operation (see, for example, Example 1). Moreover, a pure dicarboxylic acid compound can be obtained as a crystal by adjusting the conditions for crystallizing a crystal from the above liquid mixture (see, for example, Examples 2 and 3).

The liquid mixtures used in these examples (see, for example, Liquid mixture preparation examples 3 and 4) were, for example, liquid mixtures obtained by decomposing a polyester carbonate resin (see, for example, Liquid mixture preparation examples 1 and 2), and thus it was also confirmed that it is possible to recover and reuse a dicarboxylic acid compound or a dihydroxy compound from a polyester carbonate resin contained in a waste resin composition, etc. By utilizing such a method for producing a monomer compound, it is also possible to reuse a resin composition from a waste material containing a polyester resin, a polycarbonate resin, etc., and recycle a dicarboxylic acid compound, a dihydroxy compound, etc. that were used as feedstocks.

The monomer compound recycled as above can also be polymerized again to produce a recycled resin. In particular, a monomer compound obtained as a pure crystal does not require further purification step and thus allows an efficient polymerization reaction.

## Claims

1. A method for producing a dicarboxylic acid compound represented by general formula (1-1) below or general formula (1-2) below, the method comprising:

   a crystallization step in which at least the dicarboxylic acid compound is crystallized from a liquid mixture containing the dicarboxylic acid compound and a dihydroxy compound represented by general formula (2-1) below or general formula (2-2) below:

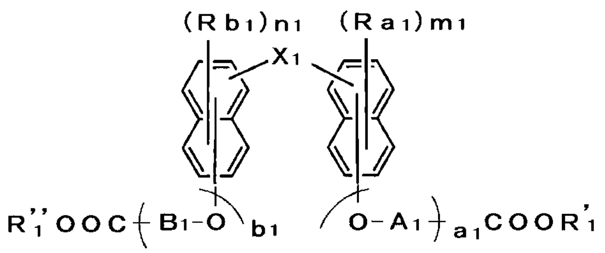

(1-1)

in general formula (1-1),
$R_{a1}$ and $R_{b1}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h1}$,
$R_{h1}$ represents an optionally substituted aryl group with 6-20 carbon atoms or an optionally substituted heteroaryl group with 6-20 carbon atoms containing one or more heteroatoms selected from O, N, and S,
$X_1$ represents a single bond or an optionally substituted fluorene group,
$A_1$ and $B_1$ each independently represent an optionally substituted alkylene group with 1-5 carbon atoms,
$m_1$ and $n_1$ each independently represent an integer from 0 to 6,
$a_1$ and $b_1$ each independently represent an integer from 0 to 10, and
$R_1$' and $R_1$" are each independently selected from the group consisting of a hydrogen atom, an optionally substituted alkyl group with 1-20 carbon atoms, and an optionally substituted aryl group with 6-20 carbon atoms;

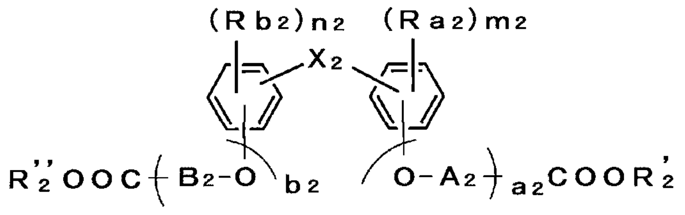

(1-2)

in general formula (1-2),

$R_{a2}$ and $R_{b2}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C\equiv C-R_{h2}$,

$R_{h2}$ represents an optionally substituted aryl group with 6-20 carbon atoms or an optionally substituted heteroaryl group with 6-20 carbon atoms containing one or more heteroatoms selected from O, N, and S,

$X_2$ is a single bond, an optionally substituted fluorene group, or any of the structural formulae represented by formulae (8)-(15) below:

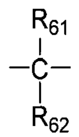

(8)

$-S-$

(9)

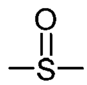

(10)

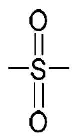

(11)

$-(CH_2)_r-$

(12)

$-O-$

(13)

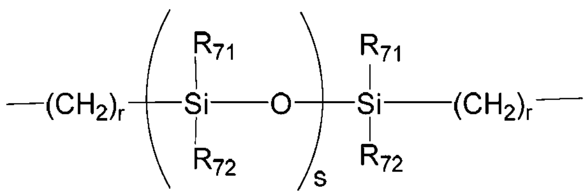

(14)

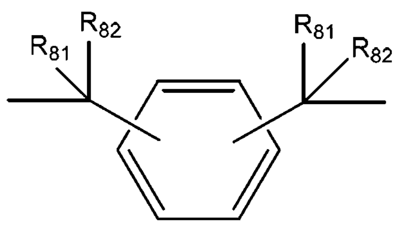

(15)

in formulae (8)-(15),

$R_{61}$, $R_{62}$, $R_{71}$, $R_{72}$, $R_{81}$, and $R_{82}$ each independently represent a hydrogen atom, a halogen atom, an optionally substituted alkyl group with 1-20 carbon atoms, or an optionally substituted aryl group with 6-30 carbon atoms, or $R_{61}$ and $R_{62}$ or $R_{71}$ and $R_{72}$ bond to each other to form an optionally substituted carbon or heterocyclic ring with 1-20 carbon atoms,

$A_2$ and $B_2$ each independently represent an optionally substituted alkylene group with 1-5 carbon atoms,

$m_2$ and $n_2$ each independently represent an integer from 0 to 6,

$a_2$ and $b_2$ each independently represent an integer from 0 to 10, and

$R_2'$ and $R_2''$ are each independently selected from the group consisting of a hydrogen atom, an optionally substituted alkyl group with 1-20 carbon atoms, and an optionally substituted aryl group with 6-20 carbon atoms;

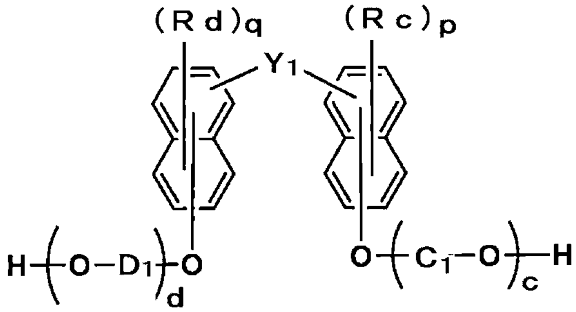

(2-1)

in general formula (2-1),

$R_c$ and $R_d$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and $-C{\equiv}C-R_{h3}$,

$R_{h3}$ represents an optionally substituted aryl group with 6-20 carbon atoms or an optionally substituted heteroaryl group with 6-20 carbon atoms containing one or more heteroatoms selected from O, N, and S,

$Y_1$ represents a single bond or an optionally substituted fluorene group,

$C_1$ and $D_1$ each independently represent an optionally substituted alkylene group with 1-5 carbon atoms,

p and q each independently represent an integer from 0 to 6, and

c and d each independently represent an integer from 0 to 10; and

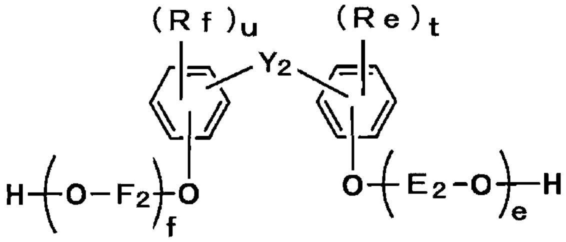

(2-2)

in general formula (2-2),

$R_e$ and $R_f$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms,

$Y_2$ is a single bond, an optionally substituted fluorene group, or any of the structural formulae represented by formulae (8)-(15) below:

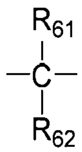

(8)

$-S-$

(9)

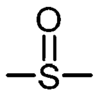

(10)

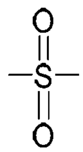

(11)

$-(CH_2)_r-$

(12)

$-O-$

(13)

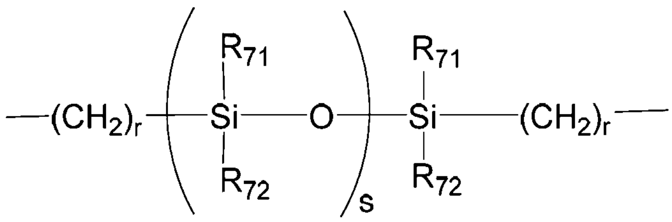

(14)

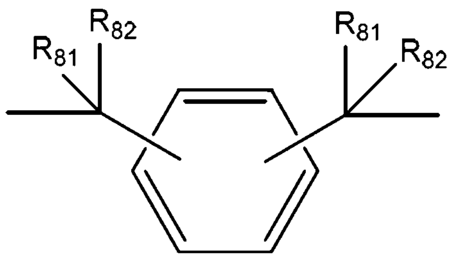

(15)

in formulae (8)-(15),

$R_{61}$, $R_{62}$, $R_{71}$, $R_{72}$,, $R_{81}$, and $R_{82}$ each independently represent a hydrogen atom, a halogen atom, an optionally substituted alkyl group with 1-20 carbon atoms, or an optionally substituted aryl group with 6-30 carbon atoms, or $R_{61}$ and $R_{62}$ or $R_{71}$ and $R_{72}$ bond to each other to form an optionally substituted carbon or heterocyclic ring with 1-20 carbon atoms, and

r and s each independently represent an integer from 0 to 5,000,

$E_2$ and $F_2$ each independently represent an optionally substituted alkylene group with 1-5 carbon atoms,

t and u each independently represent an integer from 0 to 4, and

e and f each independently represent an integer from 0 to 10.

2. The method for producing a dicarboxylic acid compound according to claim 1, further comprising a first washing step in which the liquid mixture containing the dicarboxylic acid compound is washed with an acidic aqueous solution.

3. The method for producing a dicarboxylic acid compound according to claim 1, further comprising a first solution forming step in which the dicarboxylic acid compound is dissolved in a polar solvent to obtain the liquid mixture.

4. The method for producing a dicarboxylic acid compound according to claim 3, wherein

in the first solution forming step, the dicarboxylic acid compound and the dihydroxy compound are dissolved in a polar solvent to obtain the liquid mixture, and

in the crystallization step, a crystal of only the dicarboxylic acid compound is crystallized from the liquid mixture.

5. The method for producing a dicarboxylic acid compound according to claim 1, further comprising a second washing step in which the liquid mixture containing the dicarboxylic acid compound is washed with an organic solvent.

6. The method for producing a dicarboxylic acid compound according to claim 1, further comprising a second solution forming step in which the dicarboxylic acid compound is dissolved in an organic solvent to obtain the liquid mixture.

7. The method for producing a dicarboxylic acid compound according to claim 6, wherein

in the second solution forming step, the dicarboxylic acid compound and the dihydroxy compound are dissolved in an organic solvent to obtain the liquid mixture, and

in the crystallization step, a co-crystal of the dicarboxylic acid compound and the dihydroxy compound is crystallized from the liquid mixture.

8. The method for producing a dicarboxylic acid compound according to claim 1, further comprising a decomposition step in which a polyester carbonate resin and/or a polyester resin, which has at least a structural unit derived from a dicarboxylic acid compound represented by general formula (1-1') below or general formula (1-2') below, is decomposed to obtain the dicarboxylic acid compound represented by general formula (1-1) above or general formula (1-2) above:

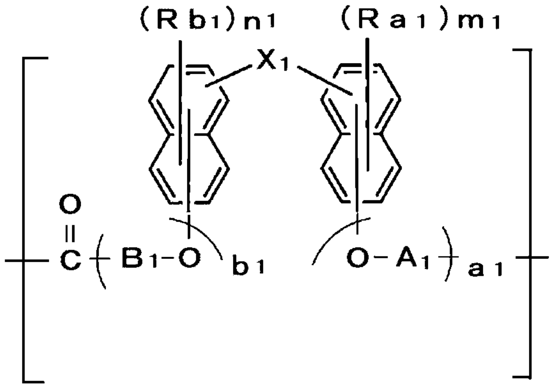

(1－1')

each symbol in general formula (1-1') is synonymous with the one in general formula (1-1) above, and

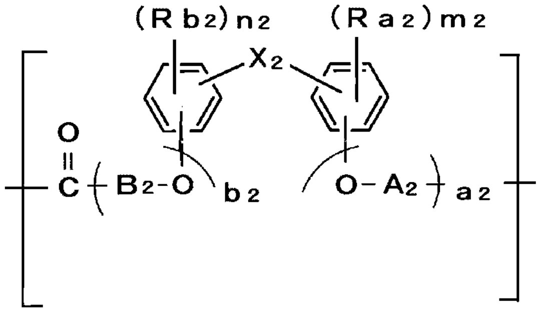

(1－2')

each symbol in general formula (1-2') is synonymous with the one in general formula (1-2) above.

**9.** The method for producing a dicarboxylic acid compound according to claim 8, wherein

in the decomposition step, the polyester carbonate resin and/or the polyester resin is dissolved in a first solvent and treated with an alkaline solution to obtain a reaction solution containing the first solvent and the dicarboxylic acid compound represented by formula (1-1) or formula (1-2) above,
in the crystallization step, the dicarboxylic acid compound is crystallized from a liquid mixture subjected to crystallization which is obtained by adding a second solvent to the reaction solution, and
the difference between the solubility of the dicarboxylic acid compound in the first solvent at 25°C and the solubility of the dicarboxylic acid compound in the second solvent at 25°C is 0.1 g/10 mL or more.

**10.** The method according to claim 9, wherein the content of the first solvent in the liquid mixture subjected to crystallization is 0.1-10 g/g relative to the total amount of the mixture of the dicarboxylic acid compound.

**11.** The method according to claim 9, wherein the content of the second solvent in the liquid mixture subjected to crystallization is 0.3-3 g/g relative to the total amount of the mixture of the dicarboxylic acid compound.

**12.** The method according to claim 9, wherein the total content of the first and second solvents in the liquid mixture subjected to crystallization is 1-10 g/g relative to the total amount of the dicarboxylic acid compound.

**13.** The method according to claim 9, wherein the ratio of the content of the first solvent (g/g) to the content of the second solvent (g/g) (first solvent/second solvent) in the liquid mixture subjected to crystallization is 0.8-10.

**14.** The method according to claim 1, wherein, in the crystallization step, a seed crystal of the dicarboxylic acid compound or the dihydroxy compound is added to the liquid mixture to crystallize a co-crystal of the dicarboxylic acid compound and the dihydroxy compound.

**15.** A method for producing a dicarboxylic acid compound represented by general formula (1-1) below or general formula (1-2) below, the method comprising:

a crystallization step in which at least the dicarboxylic acid compound is crystallized from a liquid mixture containing the dicarboxylic acid compound,

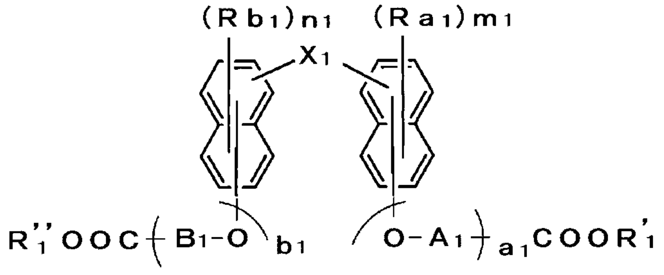

(1-1)

in general formula (1-1),
$R_{a1}$ and $R_{b1}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and -C≡C-$R_{h1}$,
$R_{h1}$ represents an optionally substituted aryl group with 6-20 carbon atoms or an optionally substituted heteroaryl group with 6-20 carbon atoms containing one or more heteroatoms selected from O, N, and S,
$X_1$ represents a single bond or an optionally substituted fluorene group,
$A_1$ and $B_1$ each independently represent an optionally substituted alkylene group with 1-5 carbon atoms,
$m_1$ and $n_1$ each independently represent an integer from 0 to 6,
$a_1$ and $b_1$ each independently represent an integer from 0 to 10, and
$R_1$' and $R_1$" are each independently selected from the group consisting of a hydrogen atom, an optionally substituted alkyl group with 1-20 carbon atoms, and an optionally substituted aryl group with 6-20 carbon atoms; and

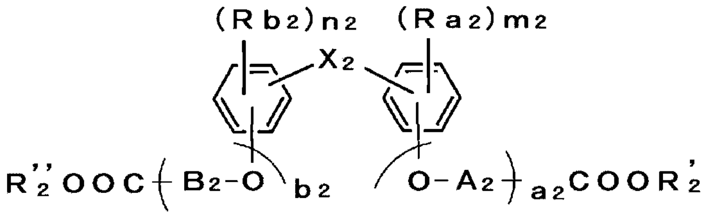

(1-2)

in general formula (1-2),
$R_{a2}$ and $R_{b2}$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N, and S, an optionally substituted aryloxy group with 6-20 carbon atoms, and -C≡C-$R_{h2}$,
$R_{h2}$ represents an optionally substituted aryl group with 6-20 carbon atoms or an optionally substituted heteroaryl group with 6-20 carbon atoms containing one or more heteroatoms selected from O, N, and S,
$X_2$ is a single bond or an optionally substituted fluorene group,
$A_2$ and $B_2$ each independently represent an optionally substituted alkylene group with 1-5 carbon atoms,
$m_2$ and $n_2$ each independently represent an integer from 0 to 6,
$a_2$ and $b_2$ each independently represent an integer from 0 to 10, and

$R_2$' and $R_2$" are each independently selected from the group consisting of a hydrogen atom, an optionally substituted alkyl group with 1-20 carbon atoms, and an optionally substituted aryl group with 6-20 carbon atoms.

**16.** A method for producing a recycled resin, comprising a polymerization step in which a dicarboxylic acid compound produced by the method according to any one of claims 1-15 is polymerized.

**INTERNATIONAL SEARCH REPORT**

International application No.
**PCT/JP2024/023955**

**A. CLASSIFICATION OF SUBJECT MATTER**

***C07C 51/43***(2006.01)i; ***C07C 27/02***(2006.01)i; ***C07C 51/48***(2006.01)i; ***C07C 59/66***(2006.01)i; ***C08G 63/64***(2006.01)i; ***C08J 11/16***(2006.01)i
FI: C07C51/43 ZAB; C07C51/48; C08J11/16; C08G63/64; C07C59/66; C07C27/02

According to International Patent Classification (IPC) or to both national classification and IPC

**B. FIELDS SEARCHED**

Minimum documentation searched (classification system followed by classification symbols)

C07C51/43; C07C27/02; C07C51/48; C07C59/66; C08G63/64; C08J11/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2015-196676 A (TAOKA CHEM. CO., LTD.) 09 November 2015 (2015-11-09) claims, paragraphs [0002], [0012], [0013], [0023], [0029], [0044], [0049], [0050], examples | 1, 3-6, 8-13, 15, 16 |
| Y | | 1, 3-6, 8-13, 15, 16 |
| A | | 2, 7, 14 |
| Y | WO 2020/137927 A1 (MITSUBISHI GAS CHEMICAL COMPANY, INC.) 02 July 2020 (2020-07-02) claims, paragraph [0009], example 4 | 1, 3-6, 8-13, 15, 16 |
| A | | 2, 7, 14 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 September 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/023955**

| Patent document cited in search report | Publication date (day/month/year) | Patent family member(s) | Publication date (day/month/year) |
|---|---|---|---|
| JP 2015-196676 A | 09 November 2015 | (Family: none) | |
| WO 2020/137927 A1 | 02 July 2020 | JP 2024-79821 A | |
| | | CN 113260883 A | |
| | | KR 10-2021-0108943 A | |
| | | TW 202037632 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011131507 A
- WO 2023058599 A